# EUROPEAN PATENT APPLICATION

(11) **EP 3 373 007 A1**
(43) Date of publication of application: **12.09.2018**
(21) Application number: 17159341.1
(22) Date of filing: 06.03.2017
(51) Int. Cl.: G01N 33/564

(54) **BIOMARKERS FOR DIAGNOSIS AND PROGRESSION OF PRIMARY PROGRESSIVE MULTIPLE SCLEROSIS (PPMS)**

(71) Applicant: Metabolomic Discoveries GmbH, 14476 Potsdam (DE); Fraunhofer-Gesellschaft zur Förderung der angewandten Forschung e.V., 80686 München (DE); Universitätsklinikum Hamburg-Eppendorf, 20246 Hamburg (DE)
(72) Inventor: Stoessel, Daniel, 13357 Berlin (DE); Schauer, Nicolas, 14476 Potsdam (DE); Pless, Ole, 22525 Hamburg (DE); Friese, Manuel, 20251 Hamburg (DE)
(74) Representative: Geling, Andrea

(57) **Abstract**

The present invention relates to a method of diagnosing primary progressive multiple sclerosis (PPMS) in a patient suspected of having PPMS. Further, the present invention relates to a method of determining the course of PPMS in a patient having PPMS. Furthermore, the present invention relates to a method of determining the severity of PPMS in a patient suspected of having PPMS. It also relates to the use of at least one metabolite for diagnosing PPMS in a patient suspected of having PPMS, for determining the course of PPMS in a patient having PPMS, or for determining the severity of PPMS in a patient suspected of having PPMS. In addition, it relates to a kit for diagnosing PPMS in a patient suspected of having PPMS, for determining the course of PPMS in a patient having PPMS, or for determining the severity of PPMS in a patient suspected of having PPMS.

## Description

The present invention relates to a method of diagnosing primary progressive multiple sclerosis (PPMS) in a patient suspected of having PPMS. Further, the present invention relates to a method of determining the course of PPMS in a patient having PPMS. Furthermore, the present invention relates to a method of determining the severity of PPMS in a patient suspected of having PPMS. It also relates to the use of at least one metabolite for diagnosing PPMS in a patient suspected of having PPMS, for determining the course of PPMS in a patient having PPMS, or for determining the severity of PPMS in a patient suspected of having PPMS. In addition, it relates to a kit for diagnosing PPMS in a patient suspected of having PPMS, for determining the course of PPMS in a patient having PPMS, or for determining the severity of PPMS in a patient suspected of having PPMS.

### BACKGROUND OF THE INVENTION

Multiple sclerosis (MS) is considered as an inflammatory and degenerative disease of the central nervous system (CNS). It shows high heterogeneity with regard to its pathology and clinical progression. In particular, no parameters exist to reliably diagnose MS, specify its subtype at diagnosis and quantify the amount of neurodegeneration during disease progression. At diagnosis, the common relapsing-remitting multiple sclerosis (RRMS; approx. 85% of cases) subtype is distinguished from primary progressive multiple sclerosis (PPMS; approx. 15% of cases). Relapsing remitting multiple sclerosis (RRMS) has a highly variable disease progression and poor prognosis (Koch *et al.,* 2009). The course of the disease rarely shows clinical relapses as present in the more common subtype of relapsing remitting multiple sclerosis (RRMS), but a progressive aggravation of disabilities is characteristic with no effective treatment options available (Koch *et al.,* 2009; Stellmann *et al.,* 2014). In addition, PPMS shows lower inflammatory activity with a rapid disease progression and fewer brain MRI lesions compared to RRMS (Fitzner and Simons, 2010; Stellmann *et al.,* 2014; Correale *et al.,* 2016). Overall the pathophysiological differences make it more difficult to diagnose and treat PPMS.

Multiple sclerosis diagnosis is currently based on clinical assessment such as the expanded disability status scale, MRI based on the McDonald criteria (Polman *et al.,* 2005, 2011) and analysis of the cerebrospinal fluid for the presence of oligoclonal bands (Compston and Coles, 2008). However, reliable diagnosis by imaging, clinical or biological markers for the individual disease course specific to PPMS is not yet possible, the progress highly heterogeneous and metabolic alterations that correlate or predict disease progression are currently unknown (Tremlett *et al.,* 2005; Stellmann *et al.,* 2014). In addition, biological markers indicating disease progression would improve our mechanistic understanding and quantification of neurodegeneration in MS and potential treatment trials (Fox *et al.,* 2012; Friese *et al.,* 2014).

Untargeted metabolomics is a rapidly evolving high-throughput technology that allows for the measurement of thousands of metabolites, typically in a mass range of 50 - 1700 Da, in complex samples such as biological fluids or tissues (Want *et al.,* 2013; Contrepois *et al.,* 2015). The application of high resolution mass spectrometry (HRMS) in combination with liquid chromatography enables simultaneous semi-quantitative measurements of various molecular species such as amino acids, nucleotides, sphingolipids, and phospholipids (Zhou *et al.,* 2012; Want *et al.,* 2013; Poisson *et al.,* 2015) and is, therefore, a powerful technology to obtain a comprehensive view of the functional state of the human organism (Smolinska *et al.,* 2012; Cocco *et al.,* 2016). In addition, the high sensitivity and reliability makes this technology suitable for risk assessment and diagnosis in a single non-invasive analysis (Floegel *et al.,* 2011).

Metabolome-based studies in MS and other neurological diseases are limited (Bhargava and Calabresi, 2016; Hassan-Smith *et al.,* 2016). However, their potential can be seen in examples such as that of Reinke and co-workers (Reinke *et al.,* 2014).

To overcome the above-described disadvantages, novel methods and approaches are needed. The present inventors applied untargeted high-resolution metabolomics to measure metabolites in biological samples in order to identify and validate metabolic markers that allow diagnosis of PPMS and monitoring of neurodegeneration over time. They identified and validated new non-invasive metabolic makers allowing the diagnosis of PPMS in a patient suspected of having PPMS. Said metabolites allow a highly sensitive and specific discrimination between PPMS patients and healthy patients with an area under the curve (AUC) value of ∼90%. In addition, the present inventors identified and validated a new non-invasive metabolic marker allowing the determination of the course of PPMS in a patient having PPMS and the severity of PPMS in a patient suspected of having PPMS.

### SUMMARY OF THE INVENTION

In a first aspect, the present invention relates to a method of diagnosing primary progressive multiple sclerosis (PPMS) in a patient suspected of having PPMS comprising the step of:
determining the level of at least one metabolite in a biological sample from a patient suspected of having PPMS,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

In a second aspect, the present invention relates to a method of determining the course of primary progressive multiple sclerosis (PPMS) in a patient having PPMS comprising the step of:
determining the level of an alkyl ether substituted PC in a biological sample from a patient having PPMS.

In a third aspect, the present invention relates to a method of determining the severity of primary progressive multiple sclerosis (PPMS) in a patient suspected of having PPMS comprising the step of:
determining the level of an alkyl ether substituted PC in a biological sample from a patient suspected of having PPMS.

In a fourth aspect, the present invention relates to the use of at least one metabolite for diagnosing primary progressive multiple sclerosis (PPMS) in a patient suspected of having PPMS,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

In a fifth aspect, the present invention relates to the use of an alkyl ether substituted PC for determining the course of primary progressive multiple sclerosis (PPMS) in a patient having PPMS or for determining the severity of PPMS in a patient suspected of having PPMS.

In a sixth aspect, the present invention relates to a kit comprising means for determining the level of at least one metabolite in a biological sample from a patient,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of" according to the present invention means the inclusion of a stated integer or group of integers, while excluding modifications or other integers which would materially affect or alter the stated integer. The term "consisting of" or variations such as "consists of" according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "multiple sclerosis (MS)", as used herein, refers to an inflammatory demyelinating disease of the central nervous system (CNS). MS is characterized by destruction of myelin, associated with death of oligodendrocytes and axonal loss. The main pathologic finding in MS is the presence of infiltrating mononuclear cells, predominantly T lymphocytes and macrophages, which surpass the blood brain barrier (BBB) and induce an active inflammation within the brain and spinal cord. The neurological symptoms that characterize MS include, but are not limited to, complete or partial vision loss, diplopia, sensory symptoms, motor weakness that can worsen to complete paralysis, bladder dysfunction and cognitive deficits, which eventually may lead to a significant disability. The associated multiple inflammatory foci can lead to myelin destruction, plaques of demyelination, gliosis and axonal loss within the brain and spinal cord. These are the reasons which contribute to the clinical manifestations of neurological disabilities. There are four main forms of multiple sclerosis: relapsing-remitting multiple sclerosis (RRMS), secondary progressive multiple sclerosis (SPMS), primary progressive multiple sclerosis (PPMS), and progressive relapsing multiple sclerosis (PRMS).

The term "relapsing-remitting multiple sclerosis (RRMS)", as used herein, refers to a MS disease which is characterized by relapses (also known as exacerbations) during which new symptoms can appear and old ones resurface or worsen. The relapses are followed by periods of remission, during which the person fully or partially recovers from the deficits acquired during the relapse. Relapses can last for days, weeks or months and recovery can be slow and gradual or almost instantaneous.

The term "secondary progressive multiple sclerosis (SPMS) (sometimes called "galloping MS")", as used herein, describes those with an initial relapsing-remitting multiple sclerosis (RRMS), who then begin to have progressive neurologic decline between acute attacks without any definite periods of remission. Occasional relapses and minor remissions may appear. The median time between disease onset and conversion from relapsing-remitting to secondary progressive MS is about 19 years.

The term "primary progressive multiple sclerosis (PPMS)", as used herein, refers to a MS disease which is characterised by a gradual progression of the disease from its onset with no remissions at all. The primary progressive subtype describes patients who never have remissions after their initial MS symptoms.

The term "progressive relapsing multiple sclerosis (PRMS)", as used herein, refers to a form of MS which follows a progressive course from onset, punctuated by relapses. There is significant recovery immediately following a relapse but between relapses there is a gradual worsening of symptoms.

The term "diagnosing primary progressive multiple sclerosis (PPMS)", as used herein, means determining whether a patient shows signs of or suffers from PPMS.

The term "determining the course of primary progressive multiple sclerosis (PPMS)", as used herein, means determining the development of PPMS over time, e.g. whether PPMS worsens in the patient, does not worsen/is stable in the patient, or improves in the patient over time.

The term "determining the severity of primary progressive multiple sclerosis (PPMS)", as used herein, means determining the degree and/or prognosis of PPMS, e.g. whether the patient has a severe form of PPMS with a poor prognosis or a mild form of PPMS with a good prognosis.

The term "patient", as used herein, refers to any subject for whom it is desired to know whether she or he suffers from primary progressive multiple sclerosis (PPMS). In particular, the term "patient", as used herein, refers to a subject suspected to be affected by PPMS. The patient may be diagnosed to be affected by PPMS, i.e. diseased, or may be diagnosed to be not affected by PPMS, i.e. healthy. The term "patient", as used herein, also refers to a subject which is affected by PPMS, i.e. diseased. The patient may be retested for PPMS and may be diagnosed to be still affected by PPMS, i.e. diseased, or not affected by PPMS anymore, i.e. healthy, for example after therapeutic intervention. The patient may also be retested for PPMS and may be diagnosed as having developed an advanced form of PPMS.
It should be noted that a patient that is diagnosed as being healthy, i.e. not suffering from PPMS, may possibly suffer from another disease not tested/known.
The patient may be any mammal, including both a human and another mammal, e.g. an animal such as a rabbit, mouse, rat, or monkey. Human patients are particularly preferred.

The term "(control) subject", as used herein, refers to a subject known to be affected by primary progressive multiple sclerosis (PPMS) (positive control), i.e. diseased. Said (control) subject may have developed an advanced form of PPMS. The term "(control) subject", as used herein, also refers to a subject known to be not affected by PPMS (negative control), i.e. healthy. Thus, the term "healthy subject", as used herein, means a subject which is known to be not affected by PPMS.
It should be noted that a (control) subject which is known to be healthy, i.e. not suffering from PPMS, may possibly suffer from another disease not tested/known.
The (control) subject may be any mammal, including both a human and another mammal, e.g. an animal such as a rabbit, mouse, rat, or monkey. Human (control) subjects are particularly preferred.

The term "treatment", in particular "therapeutic treatment", as used herein, refers to any therapy which improves the health status and/or prolongs (increases) the lifespan of a patient. Said therapy may eliminate the disease in a patient, arrest or slow the development of a disease in a patient, inhibit or slow the development of a disease in a patient, decrease the frequency or severity of symptoms in a patient, and/or decrease the recurrence in a patient who currently has or who previously has had a disease. The treatment of PPMS includes, but is not limited to, administration of a drug, exercise training, mental training, and/or physical rehabilitation.

The term "sensitivity", as used herein, refers to the number of true positive patients (%) with regard to the number of all patients (100%). The patients may be individuals having primary progressive multiple sclerosis (PPMS). The sensitivity is calculated by the following formula: Sensitivity= TP/(TP+FN) (TP= true positives; FN=false negatives).

The term "specificity", as used herein, relates to the number of true negative individuals (%) with regard to the number of all healthy subjects (100%). The specificity is calculated by the following formula: Specificity= TN/(TN+FP) (TN= true negatives; FP=false positives).

The term "accuracy", as used herein, means a statistical measure for the correctness of classification or identification of sample types. The accuracy is the proportion of true results (both true positives and true negatives).

The result of each analysis group is usually calculated from a plurality of isolated biological samples, i.e. from at least 2 isolated biological samples, preferably from between 2 and 20, more preferably from between 10 and 60, and even more preferably from between 50 and 100 isolated biological samples, selected from the group consisting of healthy subjects and patients having PPMS. The methods of the present invention can be carried out in combination with other diagnostic methods for the detection of PPMS to increase the overall sensitivity and/or specificity. The detection of the metabolites of the present invention allows the determination of PPMS.

The term "AUC", as used herein, relates to an abbreviation for the area under a curve. In particular, it refers to the area under a Receiver Operating Characteristic (ROC) curve. The term "Receiver Operating Characteristic (ROC) curve", as used herein, refers to a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve is a measure for the accuracy of a diagnostic test (the larger the area the better, optimum is 1, a random test would have a ROC curve lying on the diagonal with an area of 0.5 (see, for reference, for example, JP. Egan. Signal Detection Theory and ROC Analysis).

The term "level", as used herein, refers to an amount (measured for example in grams, mole, or ion counts) or concentration (e.g. absolute or relative concentration) of at least one metabolite selected from the group consisting of citrulline, creatinine, L-tryptophan, LysoPE, LysoPC, PE, PC, tiglylcarnitine, hydroxy-octadecatrienoic acid, and octadecatrienoic acid. Particularly, the term "level", as used herein, refers to an amount (measured for example in grams, mole, or ion counts) or concentration (e.g. absolute or relative concentration) of at least one metabolite selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z,12Z)-Octadecatrienoic acid.
The term "level", as used herein, also comprises scaled, normalized, or scaled and normalized amounts or values. The level may also be a cut-off level.

The term "biological sample", as used herein, refers to any biological sample from a patient or (control) subject containing at least one metabolite selected from the group consisting of citrulline, creatinine, L-tryptophan, LysoPE, LysoPC, PE, PC, tiglylcarnitine, hydroxy-octadecatrienoic acid, and octadecatrienoic acid. Particularly, the term "biological sample", as used herein, refers to any biological sample from a patient or (control) subject containing at least one metabolite selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z,12Z)-Octadecatrienoic acid.
The biological sample may be a body fluid sample. For example, biological samples encompassed by the present invention are blood (e.g. whole blood or blood fraction such as blood cell fraction, serum or plasma) samples, urine samples, or samples from other peripheral sources. Said biological samples may be mixed or pooled, e.g. a sample may be a mixture of a blood sample and a urine sample. Said biological samples may be provided by removing a body fluid from a patient or (control) subject, but may also be provided by using a previously isolated sample. For example, a blood sample may be taken from a patient or (control) subject by conventional blood collection techniques. The biological sample, e.g. urine sample or blood sample, may be obtained from a patient or (control) subject prior to the initiation of a therapeutic treatment, during the therapeutic treatment, and/or after the therapeutic treatment. If the biological sample is obtained from at least one (control) subject, e.g. from at least 1, 2, 3, 4, 5, 6, 7, 8, 9, 10, 20, 30, 40, 50, 60, 70, 80, 90, 100, 150, 200, 250, 300, 400, 500, or 1.000 (control) subject(s), it is designated as "reference biological sample". Preferably, the reference biological sample is from the same source than the biological sample of the patient to be tested, e.g. both are blood samples or urine samples. It is further preferred that both are from the same species, e.g. from a human. It is also (alternatively or additionally) preferred that the measurements of the reference biological sample and the biological sample of the patient to be tested are identical, e.g. both have an identical volume. It is particularly preferred that the reference biological sample and the biological sample are from patients/(control) subjects of the same sex and similar age, e.g. no more than 2 years apart from each other.

The term "body fluid sample", as used herein, refers to any liquid sample derived from the body of a patient or (control) subject containing at least one metabolite selected from the group consisting of citrulline, creatinine, L-tryptophan, LysoPE, LysoPC, PE, PC, tiglylcarnitine, hydroxy-octadecatrienoic acid, and octadecatrienoic acid. Particularly, the term "body fluid sample", as used herein, refers to any body fluid sample from a patient or (control) subject containing at least one metabolite selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z, 12Z)-Octadecatrienoic acid.
Said body fluid sample may be a urine sample, blood sample, sputum sample, breast milk sample, cerebrospinal fluid (CSF) sample, cerumen (earwax) sample, gastric juice sample, mucus sample, endolymph fluid sample, perilymph fluid sample, peritoneal fluid sample, pleural fluid sample, saliva sample, sebum (skin oil) sample, semen sample, sweat sample, tears sample, cheek swab, vaginal secretion sample, liquid biopsy, or vomit sample including components or fractions thereof. The term "body fluid sample" also encompasses body fluid fractions, e.g. blood fractions, urine fractions or sputum fractions. The body fluid samples may be mixed or pooled. Thus, a body fluid sample may be a mixture of a blood and a urine sample or a mixture of a blood and cerebrospinal fluid sample. Said body fluid sample may be provided by removing a body liquid from a patient or (control) subject, but may also be provided by using previously isolated body fluid sample material. The body fluid sample allows for a non-invasive analysis of a patient. It is further preferred that the body fluid sample has a volume of between 0.01 and 20 ml, more preferably of between 0.1 and 10 ml, even more preferably of between 0.5 and 8 ml, and most preferably of between 1 and 5 ml. If the body fluid sample is obtained from at least one (control subject), e.g. from at least 1, 2, 3, 4, 5, 6, 7, 8, 9, 10, 20, 30, 40, 50, 60, 70, 80, 90, 100, 150, 200, 250, 300, 400, 500, or 1.000 control subject(s), it is designated as "reference body fluid sample".

The term "blood sample", as used herein, encompasses a whole blood sample or a blood fraction sample such as a blood cell fraction, blood serum, or blood plasma sample. It is preferred that the blood serum or plasma sample has a volume of between 0.01 and 20 ml, more preferably of between 0.1 and 10 ml, even more preferably of between 0.5 and 8 ml and most preferably of between 1 and 5 ml.

As used herein, the expression "LysoPE" refers to lysophosphatidylethanolamine. Usually, lysophosphatidylethanolamines are composed of an ethanolamine head group, a glycerophosphoric acid, and an acyl group. In the context of the present invention, the acyl group may be replaced by an alkyl group or a 1Z-alkenyl group. The acyl group (or, if present, the alkyl group or, if present, the 1Z-alkenyl group) within the LysoPE molecule can be saturated or unsaturated. The chain length of the acyl group (or, if present, the alkyl group or, if present, the 1Z-alkenyl group) and the number of double bonds within the acyl group (or, if present, the alkyl group or, if present, the 1Z-alkenyl group) may be indicated in round brackets after the term "LysoPE"; e.g. the expression LysoPE(18:1) denotes a lysophosphatidylethanolamine with an octadecenoic acid, i.e. a C-18 fatty acid with one double bond. Examples of such an octadecenoic acid are oleic acid, elaidic acid, and vaccenic acid.

As used herein, the expression "LysoPC" refers to lysophosphatidylcholine. Usually, lysophosphatidylcholines are composed of a choline head group, a glycerophosphoric acid, and an acyl group. In the context of the present invention, the acyl group may be replaced by an alkyl group or a 1Z-alkenyl group. The acyl group (or, if present, the alkyl group or, if present, the 1Z-alkenyl group) within the LysoPC molecule can be saturated or unsaturated. The chain length of the acyl group (or, if present, the alkyl group or, if present, the 1Z-alkenyl group) and the number of double bonds within the acyl group (or, if present, the alkyl group or, if present, the 1Z-alkenyl group) may be indicated in round brackets after the term "LysoPC"; e.g. the expression LysoPC(20:1) denotes a lysophosphatidylcholine with an eicosenoic acid, i.e. a C-20 fatty acid with one double bond. Examples of such an eicosenoic acid are gadoleic acid, gondoic acid, and paullinic acid.

As used herein, the expression "PE" refers to phosphatidylethanolamine. Usually, phosphatidylethanolamines are composed of an ethanolamine head group, a glycerophosphoric acid, and two acyl groups. In the context of the present invention, the acyl groups may be replaced - independently from each other - by an alkyl group or a 1Z-alkenyl group. The acyl groups (or, if present, the alkyl groups or, if present, the 1Z-alkenyl groups) within the LysoPE molecule can - independently from each other - be saturated or unsaturated. The total chain length of the acyl groups (or, if present, the alkyl groups or, if present, the 1Z-alkenyl groups) and the total number of double bonds within the acyl groups (or, if present, the alkyl groups or, if present, the 1Z-alkenyl groups) may be indicated in round brackets after the term "PE"; e.g. the expression PE(36:5) indicates that the phophatidylethanolamine comprises two fatty acids with a total number of 36 carbon atoms and a total of five double bonds.

As used herein, the expression "PC" refers to phosphatidylcholine. Usually, phosphatidylcholines are composed of a choline head group, a glycerophosphoric acid, and two acyl groups. In the context of the present invention, the acyl groups may be replaced - independently from each other - by an alkyl group or a 1Z-alkenyl group. The acyl groups (or, if present, the alkyl groups or, if present, the 1Z-alkenyl groups) within the LysoPC molecule can - independently from each other - be saturated or unsaturated. The total chain length of the acyl groups (or, if present, the alkyl groups or, if present, the 1Z-alkenyl groups) and the total number of double bonds within the acyl groups (or, if present, the alkyl groups or, if present, the 1Z-alkenyl groups) may be indicated in round brackets after the term "PC"; e.g. the expression PC(44:12) indicates that the phophatidylethanolcholine comprises two fatty acids with a total number of 44 carbon atoms and a total of 12 double bonds.

As explained above, the numbers in round brackets following the expressions "LysoPE", "LysoPC", "PE", and "PC" indicate the total chain length of the acyl group(s) (or, if present, the alkyl group(s) or, if present, the 1Z-alkenyl group(s)) and the total number of double bonds within the acyl group(s) (or, if present, the alkyl group(s) or, if present, the 1Z-alkenyl group(s)) in each molecule. The number defining the total chain length and the number defining the total number of double bonds are separated by a colon. The information provided in round brackets may additionally contain an 'O-' or a 'P-' prefix. The 'O-' prefix is used to indicate the presence of an alkyl ether substituent e.g. PC(O-16:0/4:0), whereas the 'P-' prefix is used for the 1Z-alkenyl ether (Plasmalogen) substituent e.g. LysoPC(P-16:0).

As explained above, PE and PC contain two acyl groups (or alkyl ether groups or alkenyl-ether groups). In some embodiments, information is provided about the individual chain length of each acyl group (or alkyl ether group or alkenyl-ether group) and the number of double bonds within each acyl group (or alkyl ether group or alkenyl-ether group). This information is provided by first indicating the chain length and the number of double bonds within the first acyl group (or alkyl ether group or alkenyl-ether group), which are separated by a colon, and by secondly indicating the chain length and the number of double bonds within the second acyl group (or alkyl ether group or alkenyl-ether group), which are also separated by a colon. The information relating to the first acyl group (or alkyl ether group or alkenyl-ether group) and the information relating to the second acyl group (or alkyl ether group or alkenyl-ether group) are separated by a slash.

As used herein, the expression "2(R)-HOT" refers to (2R)-(9Z,12Z,15Z)-2-Hydroxyoctadecatri-9,12,15-enoic acid.

The term "mass spectrometry (MS)", as used herein, refers to the use of an ionization source to generate gas phase ions from a sample on a surface and detecting the gas phase ions with a mass spectrometer. The mass spectrometry may be laser desorption mass spectrometry. The term "laser desorption mass spectrometry", as used herein, refers to the use of a laser as an ionization source to generate gas phase ions from a sample on a surface and detecting the gas phase ions with a mass spectrometer. The mass spectrometry may be a matrix-assisted laser desorption/ionization mass spectrometry or MALDI. In MALDI, the analyte is typically mixed with a matrix material that, upon drying, co-crystallizes with the analyte. The matrix material absorbs energy from the energy source which otherwise would fragment the labile biomolecules or analytes. The mass spectrometry may also be a surface-enhanced laser desorption/ionization mass spectrometry or SELDI. In SELDI, the surface on which the analyte is applied plays an active role in the analyte capture and/or desorption.

The term "tandem mass spectrometry (MS/MS)", as used herein, refers to multiple rounds of mass spectrometry, usually separated by some form of molecule fragmentation. For example, one mass analyzer can isolate one analyte from many entering a mass spectrometer. A second mass analyzer then stabilizes the analyte ions while they collide with a gas, causing them to fragment by collision-induced dissociation (CID). A third mass analyzer then sorts the fragments produced from the analytes. Tandem MS can also be done in a single mass analyzer over time, as in a quadrupole ion trap. There are various methods for fragmenting molecules for tandem MS, including collision-induced dissociation (CID), electron capture dissociation (ECD), electron transfer dissociation (ETD), infrared multiphoton dissociation (IRMPD), blackbody infrared radiative dissociation (BIRD), electron-detachment dissociation (EDD) and surface-induced dissociation (SID).

The term "liquid chromatography (LC)", as used herein, refers to a separation technique in which the mobile phase is a liquid. It can be carried out either in a column or a plane. A liquid chromatography that generally utilizes very small packing particles and a relatively high pressure is referred to as high performance liquid chromatography (HPLC). In HPLC, the sample is forced by a liquid at high pressure (the mobile phase) through a column that is packed with a stationary phase composed of irregularly or spherically shaped particles, a porous monolithic layer, or a porous membrane.

The term "liquid chromatography-mass spectrometry (LC-MS, or alternatively HPLC-MS)", as used herein, refers to an analytical chemistry technique that combines the physical separation capabilities of liquid chromatography (or HPLC) with the mass analysis capabilities of mass spectrometry (MS).

In the context of the present invention, the term "kit of parts (in short: kit)" is understood to be any combination of at least some of the components identified herein, which are combined, coexisting spatially, to a functional unit, and which can contain further components. Said kit may allow point-of-care testing (POCT).

The term "point-of-care testing (POCT)", as used herein, refers to a medical diagnostic testing at or near the point of care that is the time and place of patient care. This contrasts with the historical pattern in which testing was wholly or mostly confined to the medical laboratory, which entailed sending off specimens away from the point of care and then waiting hours or days to learn the results, during which time care must continue without the desired information. Point-of-care tests are simple medical tests that can be performed at the bedside. The driving notion behind POCT is to bring the test conveniently and immediately to the patient. This increases the likelihood that the patient, physician, and care team will receive the results quicker, which allows for immediate clinical management decisions to be made. POCT is often accomplished through the use of transportable, portable, and handheld instruments and test kits. Small bench analyzers or fixed equipment can also be used when a handheld device is not available - the goal is to collect the specimen and obtain the results in a very short period of time at or near the location of the patient so that the treatment plan can be adjusted as necessary before the patient leaves.

### Embodiments of the invention

The present inventors applied untargeted high-resolution metabolomics to measure metabolites in a biological sample in order to identify and validate metabolic markers that allow diagnosis of PPMS and monitoring of neurodegeneration over time. They identified and validated new non-invasive metabolic makers allowing the diagnosis of PPMS in a patient suspected of having PPMS. Said metabolites allow a highly sensitive and specific discrimination between PPMS patients and healthy patients with an area under the curve (AUC) value of ∼90%. In addition, the present inventors identified and validated a new non-invasive metabolic marker allowing the determination of the course of PPMS in a patient having PPMS and the severity of PPMS in a patient suspected of having PPMS.

Thus, in a first aspect, the present invention relates to a method of diagnosing primary progressive multiple sclerosis (PPMS) in a patient suspected of having PPMS comprising the step of:
determining the level of at least one metabolite in a biological sample from a patient suspected of having PPMS,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

In one embodiment, the level of the at least one metabolite is compared to a reference level of said at least one metabolite. Thus, in one particular embodiment, the present invention relates to a method of diagnosing primary progressive multiple sclerosis (PPMS) in a patient suspected of having PPMS comprising the steps of:
(i) determining the level of at least one metabolite in a biological sample from a patient suspected of having PPMS,
   wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid, and
(ii) comparing the level of the at least one metabolite to a reference level of said at least one metabolite.

The above comparison allows to diagnose primary progressive multiple sclerosis (PPMS) in the patient suspected of having PPMS. The patient may be diagnosed as suffering from PMMS, i.e. being diseased, or as not suffering from PPMS, i.e. being healthy.

The reference level may be any level which allows to determine whether a patient suffers from PPMS or not. It is preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40,41,42,43,44,45,46,47,48,49,50,51,52,53,54,55,56,57,58,59,60,61,62,63,64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference biological sample(s), from at least one healthy subject, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35,36,37,38,39,40,41,42,43,44,45,46,47,48,49,50,51,52,53,54,55,56,57,58,59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 healthy subject(s). It is more preferred that the reference level is the level determined by measuring between 2 and 500 reference biological samples from between 2 and 500 healthy subjects. It is even more preferred that the reference level is determined by measuring between 50 and 500 reference biological samples from between 50 and 500 healthy subjects. It is most preferred that the reference level is determined by measuring between 100 and 500 reference biological samples from between 100 and 500 healthy subjects.

It is practicable to take one reference biological sample per subject for analysis. If additional reference biological samples are required, e.g. to determine the reference level in different reference biological samples, the same subject may be (re)tested. Said reference level may be an average reference level. It may be determined by measuring reference levels and calculating the "average" value (e.g. mean, median or modal value) thereof.

As mentioned above, the level of the at least one metabolite is compared to a reference level of said at least one metabolite. Said reference level is the level determined by measuring a reference biological sample. For example, if the level of the metabolite PC is determined in a biological sample from a patient, it is compared to a reference level of the metabolite PC determined in a reference biological sample. Alternatively, if the level of the metabolite PC and the level of the metabolite PE is determined in a biological sample from a patient, both levels are compared to the respective reference levels, i.e. the level of the metabolite PC is compared to the reference level of the metabolite PC and the level of the metabolite PE is compared to the reference level of the metabolite PE determined in a reference biological sample.

It is further preferred that the level of the at least one metabolite below the reference level indicates that the patient has PPMS. Preferably, the level of the at least one metabolite is at least 0.6-fold, more preferably at least 0.7-fold, even more preferably at least 0.8-fold, most preferably at least 0.9-fold below the reference level.

Preferably,
(i) LysoPE comprises an acyl group or an alkyl group or an 1Z-alkenyl group with 18 to 22 carbon atoms and with 0 to 4 double bonds,
   more preferably, LysoPE is selected from the group consisting of LysoPE(18:1), LysoPE(18:2), and LysoPE(22:4),
(ii) LysoPC comprises an acyl group or an alkyl group or an 1Z-alkenyl group with 16 to 20 carbon atoms and with 0 to 1 double bonds,
   more preferably, LysoPC is selected from the group consisting of LysoPC(P-16:0), LysoPC(P-18:0), LysoPC(P-18:1), and LysoPC(20:1),
(iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
   more preferably, PE is PE(36:5),
   even more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
(iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
   more preferably, PC is selected from the group consisting of PC(14:0), an alkyl ether substituted PC such as PC(O-20:0), PC(33:5), PC(35:5), PC(36:2), PC(36:3), and PC(44:12),
   even more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6), most preferably, PC is 1-(6-[3]-ladderane-hexanoyl)-2-(8-[3]-ladderane-octanyl)-sn-glycerophosphocholine,
(v) hydroxy-octadecatrienoic acid is 2(R)-HOT, and/or
(vi) octadecatrienoic acid is (6Z,9Z,12Z)-Octadecatrienoic acid.

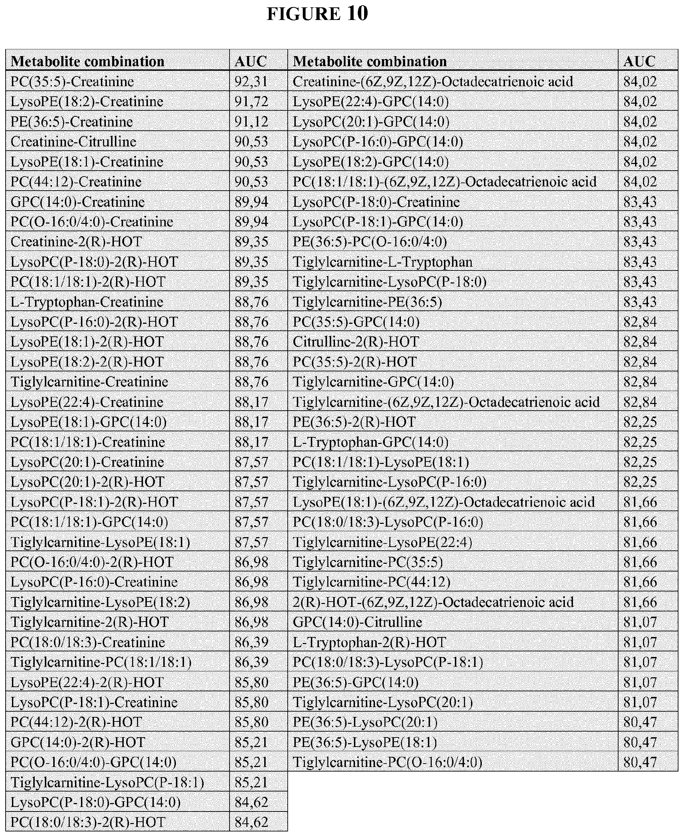

Most preferably, the at least one metabolite, e.g. the at least 1, 2, 3, 4, 5, 6, 7, 8 ,9, 10, 11, 12, 13, 14, 15, 16, 17, 18, 19, or 20 metabolite(s), is (are) selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z, 12Z)-Octadecatrienoic acid. Particularly preferred metabolite combinations (metabolite sets) with high AUC values are listed in **Figure 10****.**

It should be noted that with respect to the metabolite Citrulline, it is preferred that the level is at least 0.8 fold below the reference level. It is more preferred that the level is at least 0.9 fold below the reference level.
With respect to the metabolite Creatinine, it is preferred that the level is at least 0.8-fold below the reference level. It is more preferred that the level is at least 0.9-fold below the reference level.
With respect to the metabolite L-Tryptophan, it is preferred that the level is at least 0.8-fold below the reference level. It is more preferred that the level is at least 0.9-fold below the reference level.
With respect to the metabolite PC(O-16:0/4:0), it is preferred that the level is at least 0.7-fold below the reference level. It is more preferred that the level is at least 0.8-fold below the reference level.
With respect to the metabolite LysoPC(P-16:0), it is preferred that the level is at least 0.8-fold below the reference level.
With respect to the metabolite LysoPC(P-18:0), it is preferred that the level is at least 0.8-fold below the reference level.
With respect to the metabolite LysoPC(P-18:1), it is preferred that the level is at least 0.8-fold below the reference level.
With respect to the metabolite LysoPE(18:1), it is preferred that the level is at least 0.6-fold below the reference level. It is more preferred that the level is at least 0.7-fold below the reference level.
With respect to the metabolite LysoPE(18:2), it is preferred that the level is at least 0.6-fold below the reference level. It is more preferred that the level is at least 0.7-fold below the reference level.
With respect to the metabolite LysoPE(22:4), it is preferred that the level is at least 0.7-fold below the reference level.
With respect to the metabolite PC(18:0/18:3), it is preferred that the level is at least 0.9-fold below the reference level.
With respect to the metabolite PC(18:1/18:1), it is preferred that the level is at least 0.9-fold below the reference level.
With respect to the metabolite LysoPC(20:1), it is preferred that the level is at least 0.7-fold below the reference level. It is more preferred that the level is at least 0.8-fold below the reference level.
With respect to the metabolite PC(44:12), it is preferred that the level is at least 0.8-fold below the reference level.
With respect to the metabolite PE(36:5), it is preferred that the level is at least 0.8-fold below the reference level.
With respect to the metabolite PC(35:5), it is preferred that the level is at least 0.6-fold below the reference level. It is more preferred that the level is at least 0.7-fold below the reference level. It is even more preferred that the level is at least 0.8-fold below the reference level. With respect to the metabolite Tiglylcarnitine, it is preferred that the level is at least 0.7-fold below the reference level.
With respect to the metabolite 2(R)-HOT, it is preferred that the level is at least 0.8-fold below the reference level. It is more preferred that the level is at least 0.9-fold below the reference level.
With respect to the metabolite 1-(6-[3]-ladderane-hexanoyl)-2-(8-[3]-ladderane-octanyl)-sn-glycerophosphocholine, it is preferred that the level is at least 0.8-fold below the reference level. It is more preferred that the level is at least 0.9-fold below the reference level.
With respect to the metabolite (6Z,9Z,12Z)-Octadecatrienoic acid, it is preferred that the level is at least 0.7-fold below the reference level.

In the method of the first aspect of the present invention, it is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

Thus, in one preferred embodiment, the present invention relates to a method of diagnosing primary progressive multiple sclerosis (PPMS) in a patient suspected of having PPMS comprising the steps of:
(i) determining the level of at least one metabolite in a plasma sample from a patient suspected of having PPMS,
   wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid, and
(ii) comparing the level of the at least one metabolite to a reference level of said at least on metabolite.

In one more preferred embodiment, the present invention relates to a method of diagnosing primary progressive multiple sclerosis (PPMS) in a patient suspected of having PPMS comprising the steps of:
(i) determining the level of at least one metabolite in a plasma sample from a patient suspected of having PPMS,
   wherein the at least one metabolite is selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z,12Z)-Octadecatrienoic acid, and
(ii) comparing the level of the at least one metabolite to a reference level of said at least on metabolite.
Particularly preferred metabolite combinations (metabolite sets) with high AUC values are listed in **Figure 10**

In a second aspect, the present invention relates to a method of determining the course of primary progressive multiple sclerosis (PPMS) in a patient having PPMS comprising the step of:
determining the level of an alkyl ether substituted PC in a biological sample from a patient having PPMS.

In one embodiment, the level of the alkyl ether substituted PC is compared to a reference level of said alkyl ether substituted PC. Thus, in one particular embodiment, the present invention relates to a method comprising the steps of:
(i) determining the level of an alkyl ether substituted PC in a biological sample from a patient having PPMS, and
(ii) comparing the level of the alkyl ether substituted PC to a reference level of said alkyl ether substituted PC.

The above comparison allows to determine the course of primary progressive multiple sclerosis (PPMS) in the patient having PPMS. It may be determined that PPMS worsens in the patient, that PPMS does not worsen/is stable in the patient, or that PPMS improves in the patient.

The reference level may be any level which allows to determine the course of PPMS. In one preferred embodiment, the reference level is the level determined by measuring at least one reference biological sample from
at least one healthy subject, or
at least one subject having PPMS.
It is preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21,22,23,24,25,26,27,28,29,30,31,32,33,34,35,36,37,38,39,40,41,42,43,44,45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference biological sample(s), from at least one healthy subject, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 healthy subject(s). It is more preferred that the reference level is the level determined by measuring between 2 and 500 reference biological samples from between 2 and 500 healthy subjects. It is even more preferred that the reference level is determined by measuring between 50 and 500 reference biological samples from between 50 and 500 healthy subjects. It is most preferred that the reference level is determined by measuring between 100 and 500 reference biological samples from between 100 and 500 healthy subjects.
It is also preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference biological sample(s), from at least one subject having PPMS, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 subject(s) having PPMS. It is more preferred that the reference level is the level determined by measuring between 2 and 500 reference biological samples from between 2 and 500 subjects having PPMS. It is even more preferred that the reference level is determined by measuring between 50 and 500 reference biological samples from between 50 and 500 subjects having PPMS It is most preferred that the reference level is determined by measuring between 100 and 500 reference biological samples from between 100 and 500 subjects having PPMS.

It is practicable to take one reference biological sample per subject for analysis. If additional reference biological samples are required, e.g. to determine the reference level in different reference biological samples, the same subject may be (re)tested. Said reference level may be an average reference level. It may be determined by measuring reference levels and calculating the "average" value (e.g. mean, median or modal value) thereof.

In one alternative or additional embodiment, said determining comprises determining the level of the alkyl ether substituted PC in a biological sample at a first point in time and in at least one further biological sample at a later point in time and comparing said levels determined at the different time points. Thus, in one particular embodiment, the present invention relates to a method comprising the steps of:
(i) determining the level of an alkyl ether substituted PC in a biological sample from a patient having PPMS at a first point in time and in at least one further biological sample at a later point in time, and
(ii) comparing said levels determined at the different time points.

This proceeding allows to determine the course of PPMS in a patient having PPMS over an extended period of time, such as years.

Preferably, the level which
(i) decreases over time indicates that PPMS worsens in the patient,
(ii) does not change over time indicates that PPMS does not worsen/is stable in the patient, or
(iii) increases over time indicates that PPMS improves in the patient.
Preferably, said decrease is at least 0.9-fold over time. More preferably, said decrease is at least 1.2-fold over time. Even more preferably, said decrease is at least 1.5-fold over time. Most preferably, said decrease is a least 1.8-fold over time. For example, the decrease may be at least 0.9-fold over 1 year (12 months) and/or at least 1.8-fold over 2 years (24 months).
As mentioned above, a level which does not change over time indicates that PPMS does not worsen/is stable in the patient. "Stable" in this respect may mean that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Stable" in this respect may also mean that the detected level variation is within the accuracy of a measurement. The accuracy of a measurement depends on the measurement method used. Preferably, the level is constant over time.
As mentioned above, the detection of an increase of the level over time indicates that PPMS improves in the patient. Preferably, said increase is at least 0.9-fold over time. More preferably, said increase is at least 1.2-fold over time. Even more preferably, said increase is at least 1.5-fold over time. Most preferably, said increase is a least 1.8-fold over time. For example, the increase may be at least 0.9-fold over 1 year (12 months) and/or at least 1.8-fold over 2 years (24 months).
The time period between the first point in time and the later point(s) in time preferably amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least 12 months (1 year), at least 24 months (2 years), at least 3 years, at least 4 years, at least 5 years, at least 6 years, at least 7 years, at least 8 years, at least 9 years, or at least 10 years. For example, the patient may be routinely checked, e.g. once or twice a year. The patient may be (re)tested at 2,3,4,5,67,8,9, or 10 time points (first point in time and further point(s) in time).

In addition to the determination of the course of PPMS, the treatment of this disease can be monitored. It is namely preferred that the patient receives or has received a treatment, in particular therapeutic treatment, of PPMS during the determination of the course of PPMS. The treatment of PPMS may be selected from the group consisting of the administration of a drug, exercise training, mental training, and physical rehabilitation.
Preferably,
(i) the patient receives or has received a treatment for PPMS and the level which increases over time indicates that the patient responds to the treatment,
(ii) the patient receives or has received a treatment for PPMS and the level which does not change over time indicates that the patient does not respond to said treatment or that the disease is stable in the patient due to the treatment, or
(iii) the patient receives or has received a treatment for PPMS and the level which decreases over time indicates that the patient does not respond to said treatment.
The patient may receive a treatment during the complete determination/monitoring process (e.g. the administration of a drug) or may receive a treatment before, at, or after a first point in time (e.g. the administration of a drug) and may be retested at a later point in time. In particular, said first point in time may be before the initiation of a treatment and said later point in time may be during the treatment and/or after the treatment. If the treatment encompasses the administration of a drug and the patient responds to said treatment, the drug administration may be continued, the dose of the drug may be reduced, or the drug administration may be stopped. If the treatment encompasses the administration of a drug and the patient does not respond to said treatment, the dose of the drug may be increased, the drug may be changed, or the therapy mode may be changed, e.g. from drug administration to exercise training, mental training or physical rehabilitation.

Preferably, the alkyl ether substituted PC is PC(O-20:0), more preferably PC(O-16:0/4:0).

In the method of the second aspect of the present invention, it is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

Thus, in one preferred embodiment, the present invention relates to a method of determining the course of PPMS in a patient having PPMS comprising the steps of:
(i) determining the level of PC(O-20:0), preferably of PC(O-16:0/4:0), in a plasma sample from a patient having PPMS, and
(ii) comparing the level of PC(O-20:0), preferably of PC(O-16:0/4:0), to a reference level of said PC(O-20:0), preferably of said PC(O-16:0/4:0),.

Thus, in one another preferred embodiment, the present invention relates to a method of determining the course of PPMS in a patient having PPMS comprising the steps of:
(i) determining the level of PC(O-20:0), preferably of PC(O-16:0/4:0), in a plasma sample from a patient having PPMS at a first point in time and in at least one further plasma sample at a later point in time, and
(ii) comparing said levels determined at the different time points.

In a third aspect, the present invention relates to a method of determining the severity of primary progressive multiple sclerosis (PPMS) in a patient suspected of having PPMS comprising the step of:
determining the level of an alkyl ether substituted PC in a biological sample from a patient suspected of having PPMS.

In one embodiment, the level of the alkyl ether substituted PC is compared to a reference level of said alkyl ether substituted PC. Thus, in one particular embodiment, the present invention relates to a method of determining the severity of primary progressive multiple sclerosis (PPMS) in a patient suspected of having PPMS comprising the steps of:
(i) determining the level of an alkyl ether substituted PC in a biological sample from a patient suspected of having PPMS, and
(ii) comparing the level of the alkyl ether substituted PC to a reference level of said alkyl ether substituted PC.

The above comparison allows to determine the severity of primary progressive multiple sclerosis (PPMS) in the patient suspected of having PPMS. The patient suspected of having PPMS may be a patient having PPMS. It may be determined that the patient has a severe form of PPMS with a poor prognosis or that the patient has a mild form of PPMS with a good prognosis.

The reference level may be any level which allows to determine the severity of PPMS in a patient suspected of having PPMS. In one preferred embodiment, the reference level is the level determined by measuring at least one reference biological sample from
at least one healthy subject, or
at least one subject having PPMS.
It is preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference biological sample(s), from at least one healthy subject, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 healthy subject(s). It is more preferred that the reference level is the level determined by measuring between 2 and 500 reference biological samples from between 2 and 500 healthy subjects. It is even more preferred that the reference level is determined by measuring between 50 and 500 reference biological samples from between 50 and 500 healthy subjects. It is most preferred that the reference level is determined by measuring between 100 and 500 reference biological samples from between 100 and 500 healthy subjects.
It is also preferred that the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference biological sample(s), from at least one subject having PPMS, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 subject(s) having PPMS. It is more preferred that the reference level is the level determined by measuring between 2 and 500 reference biological samples from between 2 and 500 subjects having PPMS. It is even more preferred that the reference level is determined by measuring between 50 and 500 reference biological samples from between 50 and 500 subjects having PPMS It is most preferred that the reference level is determined by measuring between 100 and 500 reference biological samples from between 100 and 500 subjects having PPMS.

It is practicable to take one reference biological sample per subject for analysis. If additional reference biological samples are required, e.g. to determine the reference level in different reference biological samples, the same subject may be (re)tested. Said reference level may be an average reference level. It may be determined by measuring reference levels and calculating the "average" value (e.g. mean, median or modal value) thereof.

Preferably,
the level of the alkyl ether substituted PC below the reference level (determined by measuring at least one reference biological sample from at least one subject having PPMS) indicates that the patient has a severe form of PPMS with a poor prognosis, or
the level of the alkyl ether substituted PC above the reference level (determined by measuring at least one reference biological sample from at least one subject having PPMS) indicates that the patient has a mild form of PPMS with a good prognosis.

It is preferred that the level of the alkyl ether substituted PC is at least 0.7 below/above said reference level. It is more preferred that the level of the alkyl ether substituted PC is at least 0.8-fold below/above said reference level. It is even more preferred that the level of the alkyl ether substituted PC is at least 0.9-fold below/above said reference level. It is most preferred that the level of the alkyl ether substituted PC is at least 1.2-fold or at least 1.5-fold below/above the reference level.

Preferably, the alkyl ether substituted PC is PC(O-20:0), more preferably PC(O-16:0/4:0).

In the method of the third aspect of the present invention, it is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

Thus, in one preferred embodiment, the present invention relates to a method of determining the severity of PPMS in a patient suspected of having, preferably of having PPMS, comprising the steps of:
(i) determining the level of an alkyl ether substituted PC in a plasma sample from a patient suspected of having PPMS, preferably of having PPMS, and
(ii) comparing the level of the alkyl ether substituted PC to a reference level of said alkyl ether substituted PC.

Thus, in one more preferred embodiment, the present invention relates to a method of determining the severity of PPMS in a patient suspected of having, preferably of having PPMS, comprising the steps of:
(i) determining the level of PC(O-20:0), preferably of PC(O-16:0/4:0), in a plasma sample from a patient suspected of having PPMS, preferably having PPMS, and
(ii) comparing the level of PC(O-20:0), preferably of PC(O-16:0/4:0), to a reference level of said PC(O-20:0), preferably of said PC(O-16:0/4:0).

In the methods of the first to third aspect of the present invention, the patient may be a mammal. Preferably, the patient is a human.

In the methods of the first to third aspect of the present invention, it is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.
Preferably, the aforementioned biological samples are pre-treated before they are used in the methods of the first to third aspect of the present invention. Said pre-treatment may include treatments required to separate the at least one metabolite described herein, or to remove excessive material or waste. Furthermore, pre-treatments may aim at sterilizing biological samples and/or removing contaminants such as undesired cells, bacteria or viruses. Suitable techniques comprise centrifugation, extraction, fractioning, ultrafiltration, protein precipitation followed by filtration and purification and/or enrichment of compounds. Moreover, other pre-treatments are carried out in order to provide the at least one metabolite described herein in a form or concentration suitable for analysis. For example, if gas-chromatography coupled mass spectrometry is used, it will be required to derivatize the at least one metabolite described herein prior to said gas chromatography, or if liquid chromatography coupled mass spectrometry is used, it may be required to derivatize the at least one metabolite described herein prior to said liquid chromatography.

In the first to third aspect of the present invention, the level of the at least one metabolite described herein may be determined by spectrometry, chromatography, an enzymatic method, an immunochemical method, a gravimetric method, a chemosensoric method, or a combination thereof.
Suitable techniques include all chromatographic separation techniques such as liquid chromatography (LC), high performance liquid chromatography (HPLC), gas chromatography (GC), thin layer chromatography, or size exclusion or affinity chromatography. These techniques are well known in the art and can be applied by the person skilled in the art without further ado. Suitable devices for such determination are also well known in the art. For example, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). As an alternative or in addition to mass spectrometry techniques, the following techniques may be used for determination: nuclear magnetic resonance (NMR), magnetic resonance imaging (MRI), Fourier transform infrared analysis (FT-IR), ultraviolet (UV) spectroscopy, refraction index (Rl), fluorescent detection, radiochemical detection, electrochemical detection, light scattering (LS), dispersive Raman spectroscopy or flame ionisation detection (FID). These techniques are well known to the person skilled in the art and can be applied without further ado. The methods of the present invention shall be, preferably, assisted by automation. For example, biological sample processing or pre-treatment can be automated by robotics. Data processing and comparison is, preferably, assisted by suitable computer programs and databases. Automation allows using the methods of the present invention in high-throughput approaches.
Moreover, the level of the at least one metabolite described herein may also be determined by a specific chemical or biological assay. Said assay shall comprise means which allow to specifically detect the level of the at least one metabolite described herein in the biological sample. Preferably, said means are capable of specifically recognizing the chemical structure of the at least one metabolite described herein, or are capable of specifically identifying the at least one metabolite described herein on its capability to react with other compounds or its capability to elicit a response in a biological read out system (e.g., induction of a reporter gene). Means which are capable of specifically recognizing the chemical structure of the at least one metabolite described herein are, preferably, antibodies or other proteins which specifically interact with chemical structures, such as receptors or enzymes. Said antibodies include both polyclonal and monoclonal antibodies, as well as fragments thereof, such as Fv, Fab and F(ab)2 fragments that are capable of binding the antigen or hapten. Suitable proteins which are capable of specifically recognizing the at least one metabolite described herein are, preferably, enzymes which are involved in the conversion of said at least one metabolite. Said enzymes may use the at least one metabolite described herein as a substrate. Moreover, said antibodies may be used as a basis to generate oligopeptides which specifically recognize the at least one metabolite described herein. These oligopeptides shall, for example, comprise the enzyme's binding domains or pockets for the at least one metabolite described herein. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the at least one metabolite described herein may also be determined based on its capability to react with other molecules, i.e. by a specific chemical reaction. Further, the at least one metabolite described herein may be determined in a biological sample due to its capability to elicit a response in a biological read out system. The biological response shall be detected as read out indicating the presence and/or the amount of the at least one metabolite described herein comprised in the biological sample. Preferably, the determination of the at least one metabolite described herein is a quantitative process, e.g. allowing also the determination of the amount of the at least one metabolite described herein in the biological sample. How to apply these techniques is well known to the person skilled in the art. Moreover, suitable devices are commercially available.
As mentioned above, the level of one or more metabolites described herein may be determined by spectrometry and/or chromatography. Preferably,
(i) the spectrometry is mass spectrometry (MS), more preferably tandem mass spectrometry (MS/MS),
(ii) the chromatography is liquid chromatography (LC), gas chromatography (GC), or affinity chromatography, or
(iii) the chromatography is combined with spectrometry, more preferably mass spectrometry (MS), and is even preferably liquid chromatography-mass spectrometry (LC-MS) and most preferably liquid chromatography-tandem mass spectrometry (LC-MS/MS).
The biological sample used in the method of the first to third aspect of the present invention may have undergone chromatographic or other chemical processing before entering the mass spectrometer. The level of the at least one metabolite may also be determined by an immunoassay. Preferably, the immunoassay is an enzyme immunoassay, preferably an enzyme-linked immunosorbent assay (ELISA), a Western Blot (immunoblot), a radio immunoassay (RIA), or a luminescence immunoassay (LIA).

All methods of the present invention may also be designated as *in vitro* methods.

In a fourth aspect, the present invention relates to the use of at least one metabolite for diagnosing primary progressive multiple sclerosis (PPMS) in a patient suspected of having PPMS,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

The patient may be diagnosed as suffering from PPMS, i.e. being diseased, or as not suffering from PPMS, i.e. being healthy. The patient may be a mammal, preferably a human.

In order to diagnose whether a patient suspected of having PPMS suffers from PPMS or not, the level of the at least one metabolite selected from the group consisting of citrulline, creatinine, L-tryptophan, LysoPE, LysoPC, PE, PC, tiglylcarnitine, hydroxy-octadecatrienoic acid, and octadecatrienoic acid is determined in a biological sample from the patient.

It is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

Preferably,
(i) LysoPE comprises an acyl group or an alkyl group or an 1Z-alkenyl group with 18 to 22 carbon atoms and with 0 to 4 double bonds,
   more preferably, LysoPE is selected from the group consisting of LysoPE(18:1), LysoPE(18:2), and LysoPE(22:4),
(ii) LysoPC comprises an acyl group or an alkyl group or an 1Z-alkenyl group with 16 to 20 carbon atoms and with 0 to 1 double bonds,
   more preferably, LysoPC is selected from the group consisting of LysoPC(P-16:0), LysoPC(P-18:0), LysoPC(P-18:1), and LysoPC(20:1),
(iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
   more preferably, PE is PE(36:5),
   even more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
(iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
   more preferably, PC is selected from the group consisting of PC(14:0), an alkyl ether substituted PC such as PC(O-20:0), PC(33:5), PC(35:5), PC(36:2), PC(36:3), and PC(44:12),
   even more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6), most preferably, PC is 1-(6-[3]-ladderane-hexanoyl)-2-(8-[3]-ladderane-octanyl)-sn-glycerophosphocholine,
(v) hydroxy-octadecatrienoic acid is 2(R)-HOT, and/or
(vi) octadecatrienoic acid is (6Z,9Z,12Z)-Octadecatrienoic acid.

Most preferably, the at least one metabolite, e.g. the at least 1, 2, 3, 4, 5, 6, 7, 8 ,9, 10, 11, 12, 13, 14, 15, 16, 17, 18, 19, or 20 metabolite(s), is (are) selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z, 12Z)-Octadecatrienoic acid. Particularly preferred metabolite combinations (metabolite sets) with high AUC values are listed in **Figure 10****.**

Regarding the diagnosis of PPMS in the patient suspected of having PPMS, it is referred to the first aspect of the present invention.

In a fifth aspect, the present invention relates to the use of an alkyl ether substituted PC for determining the course of primary progressive multiple sclerosis (PPMS) in a patient having PPMS or for determining the severity of PPMS in a patient suspected of having PPMS.

In one embodiment, the present invention relates to the use of an alkyl ether substituted PC for determining the course of primary progressive multiple sclerosis (PPMS) in a patient having PPMS.

It may be determined that PPMS worsens in the patient, that PPMS does not worsen/is stable in the patient, or that PPMS improves in the patient. The patient may be a mammal, preferably a human.

In order to determine the course of PPMS in the patient having PPMS, the level of an alkyl ether substituted PC is determined in a biological sample from the patient.

It is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

Preferably, the alkyl ether substituted PC is PC(O-20:0), more preferably PC(O-16:0/4:0).

Regarding the determination of the course of PPMS in the patient having PPMS, it is referred to the second aspect of the present invention.

In one alternative embodiment, the present invention relates to the use of an alkyl ether substituted PC for determining the severity of PPMS in a patient suspected of having PPMS. The patient suspected of having PPMS may be a patient having PPMS. It may be determined that the patient has a severe form of PPMS with a poor prognosis or that the patient has a mild form of PPMS with a good prognosis. The patient may be a mammal, preferably a human.

In order to determine the severity of PPMS in a patient suspected of having PPMS, the level of an alkyl ether substituted PC is determined in a biological sample from the patient.

It is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

Preferably, the alkyl ether substituted PC is PC(O-20:0), more preferably PC(O-16:0/4:0).

Regarding the determination of the severity of PPMS in the patient suspected of having PPMS, it is referred to the third aspect of the present invention.

In a sixth aspect, the present invention relates to a kit comprising means for determining the level of at least one metabolite in a biological sample from a patient,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

Preferably,
(i) LysoPE comprises an acyl group or an alkyl group or an 1Z-alkenyl group with 18 to 22 carbon atoms and with 0 to 4 double bonds,
   more preferably, LysoPE is selected from the group consisting of LysoPE(18:1), LysoPE(18:2), and LysoPE(22:4),
(ii) LysoPC comprises an acyl group or an alkyl group or an 1Z-alkenyl group with 16 to 20 carbon atoms and with 0 to 1 double bonds,
   more preferably, LysoPC is selected from the group consisting of LysoPC(P-16:0), LysoPC(P-18:0), LysoPC(P-18:1), and LysoPC(20:1),
(iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
   more preferably, PE is PE(36:5),
   even more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
(iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
   more preferably, PC is selected from the group consisting of PC(14:0), an alkyl ether substituted PC such as PC(O-20:0), PC(33:5), PC(35:5), PC(36:2), PC(36:3), and PC(44:12),
   even more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6), most preferably, PC is 1-(6-[3]-ladderane-hexanoyl)-2-(8-[3]-ladderane-octanyl)-sn-glycerophosphocholine,
(v) hydroxy-octadecatrienoic acid is 2(R)-HOT, and/or
(vi) octadecatrienoic acid is (6Z,9Z,12Z)-Octadecatrienoic acid.

Most preferably, the at least one metabolite, e.g. the at least 1, 2, 3, 4, 5, 6, 7, 8 ,9, 10, 11, 12, 13, 14, 15, 16, 17, 18, 19, or 20 metabolite(s), is (are) selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z, 12Z)-Octadecatrienoic acid. Particularly preferred metabolite combinations (metabolite sets) with high AUC values are listed in **Figure 10****.**

The means for determining the level of the at least one metabolite in the biological sample from a patient encompass dipstrips or dipsticks, e.g. urine or blood dipstrips or dipsticks. Said means are tools used to determine changes in patient's urine or blood. A dipstrip or dipstick comprises different chemical pads or reagents which react (e.g. change color, in particular by applying an immune assay) when immersed in (e.g. blood or urine), and then removed from the biological sample (e.g. urine or blood sample). The result can be read after a few minutes, preferably after a few seconds. Specific means for determining the level of the at least one metabolite in a biological sample from a patient are also described above in the context of the first to third aspect of the present invention.

The patient may be a patient suspected of having PPMS or a patient having PPMS.

It is preferred that the biological sample is a body fluid sample. Preferably, the body fluid sample is selected from the group consisting of a blood sample, a urine sample, and a combination thereof. More preferably, the blood sample is a whole blood sample or a blood fraction sample. Even more preferably, the blood fraction sample is a blood cell fraction sample, a blood serum sample, or a blood plasma sample. Most preferably, the biological sample is a blood plasma sample.

The kit may be used for conducting the methods according to the first, second, and/or third aspect of the present invention. Regarding the diagnosis of PPMS in a patient suspected of having PPMS, it is referred to the first aspect of the present invention. Further, regarding the determination of the course of PPMS in a patient having PPMS, it is referred to the second aspect of the present invention. Furthermore, regarding the determination of the severity of PPMS in a patient suspected of having PPMS, it is referred to the third aspect of the present invention.

In one embodiment, the present invention relates to a kit for diagnosing primary progressive multiple sclerosis (PPMS) in a patient suspected of having PPMS comprising means for determining the level of at least one metabolite in a biological sample from a patient suspected of having PPMS,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid. Also encompassed by the present invention is the use of said kit for diagnosing PPMS in a patient suspected of having PPMS.

Preferably,
(i) LysoPE comprises an acyl group or an alkyl group or an 1Z-alkenyl group with 18 to 22 carbon atoms and with 0 to 4 double bonds,
   more preferably, LysoPE is selected from the group consisting of LysoPE(18:1), LysoPE(18:2), and LysoPE(22:4),
(ii) LysoPC comprises an acyl group or an alkyl group or an 1Z-alkenyl group with 16 to 20 carbon atoms and with 0 to 1 double bonds,
   more preferably, LysoPC is selected from the group consisting of LysoPC(P-16:0), LysoPC(P-18:0), LysoPC(P-18:1), and LysoPC(20:1),
(iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
   more preferably, PE is PE(36:5),
   even more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
(iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
   more preferably, PC is selected from the group consisting of PC(14:0), an alkyl ether substituted PC such as PC(O-20:0), PC(33:5), PC(35:5), PC(36:2), PC(36:3), and PC(44:12),
   even more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6), most preferably, PC is 1-(6-[3]-ladderane-hexanoyl)-2-(8-[3]-ladderane-octanyl)-sn-glycerophosphocholine,
(v) hydroxy-octadecatrienoic acid is 2(R)-HOT, and/or
(vi) octadecatrienoic acid is (6Z,9Z,12Z)-Octadecatrienoic acid.

Most preferably, the at least one metabolite, e.g. the at least 1, 2, 3, 4, 5, 6, 7, 8 ,9, 10, 11, 12, 13, 14, 15, 16, 17, 18, 19, or 20 metabolite(s), is (are) selected from the group consisting of Citrulline, Creatinine, L-Tryptophan, PC(O-16:0/4:0), LysoPC(P-16:0), LysoPC(P-18:0), LysoPC(P-18:1), LysoPE(18:1), LysoPE(18:2), LysoPE(22:4), PC(18:0/18:3), PC(18:1/18:1), LysoPC(20:1), PC(44:12), PE(36:5), PC(35:5), Tiglylcarnitine, 2(R)-HOT, 1-(6-[3]-ladderane-hexanoyl)-2-(8-[3]-ladderane-octanyl)-sn-glycerophosphocholine, and (6Z,9Z, 12Z)-Octadecatrienoic acid. Particularly preferred metabolite combinations (metabolite sets) with high AUC values are listed in **Figure 10****.**

In one another embodiment, the present invention relates to a kit for determining the course of primary progressive multiple sclerosis (PPMS) in a patient having PPMS comprising means for determining the level of an alkyl ether substituted PC in a biological sample from a patient having PPMS. Also encompassed by the present invention is the use of said kit for determining the course of PPMS in a patient having PPMS.

Preferably, the alkyl ether substituted PC is PC(O-20:0), more preferably PC(O-16:0/4:0).

In one another embodiment, the present invention relates to a kit for determining the severity of primary progressive multiple sclerosis (PPMS) in a patient suspected of having PPMS comprising means for determining the level of an alkyl ether substituted PC in a biological sample from a patient suspected of having PPMS. Also encompassed by the present invention is the use of said kit for determining the severity of PPMS in a patient suspected of having PPMS.

Preferably, the alkyl ether substituted PC is PC(O-20:0), more preferably PC(O-16:0/4:0).

The kit may further comprise
(i) a container, and/or
(ii) a data carrier.
Said data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.
Said data carrier may further comprise a reference level of the at least one metabolite referred to herein.
In case that the data carrier comprises an access code which allows the access to a database, said reference level is deposited in this database.
In addition, the data carrier may comprise information or instructions on how to carry out the methods according to the first, second, and/or third aspect of the present invention.

Said kit may also comprise materials desirable from a commercial and user standpoint including a buffer(s), a reagent(s) and/or a diluent(s) for determining the level mentioned above.

The invention is further summarized as follows:
1. A method of diagnosing primary progressive multiple sclerosis (PPMS) in a patient suspected of having PPMS comprising the step of:
   determining the level of at least one metabolite in a biological sample from a patient suspected of having PPMS,
   wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.
2. The method of item 1, wherein the level of the at least one metabolite is compared to a reference level of said at least one metabolite.
3. The method of item 2, wherein the reference level is the level determined by measuring at least one reference biological sample from at least one healthy subject.
4. The method of item 3, wherein the level of the at least one metabolite below the reference level indicates that the patient has PPMS.
5. The method of any one of items 1 to 4, wherein
   (i) LysoPE comprises an acyl group or an alkyl group or an 1Z-alkenyl group with 18 to 22 carbon atoms and with 0 to 4 double bonds,
      preferably, LysoPE is selected from the group consisting of LysoPE(18:1), LysoPE(18:2), and LysoPE(22:4),
   (ii) LysoPC comprises an acyl group or an alkyl group or an 1Z-alkenyl group with 16 to 20 carbon atoms and with 0 to 1 double bonds,
      preferably, LysoPC is selected from the group consisting of LysoPC(P-16:0), LysoPC(P-18:0), LysoPC(P-18:1), and LysoPC(20:1),
   (iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
      preferably, PE is PE(36:5),
      more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
   (iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
      preferably, PC is selected from the group consisting of PC(14:0), PC(O-20:0), PC(33:5), PC(35:5), PC(36:2), PC(36:3), and PC(44:12),
      more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6),
      even more preferably, PC is 1-(6-[3]-ladderane-hexanoyl)-2-(8-[3]-ladderane-octanyl)-sn-glycerophosphocholine,
   (v) hydroxy-octadecatrienoic acid is 2(R)-HOT, and/or
   (vi) octadecatrienoic acid is (6Z,9Z,12Z)-Octadecatrienoic acid.
6. A method of determining the course of primary progressive multiple sclerosis (PPMS) in a patient having PPMS comprising the step of:
   determining the level of an alkyl ether substituted PC in a biological sample from a patient having PPMS.
7. The method of item 6, wherein the level of the alkyl ether substituted PC is compared to a reference level of said alkyl ether substituted PC.
8. The method of item 7, wherein the reference level is the level determined by measuring
   at least one reference biological sample from
   at least one healthy subject, or
   at least one subject having PPMS.
9. The method of any one of items 6 to 8, wherein said determining comprises determining
   the level of the alkyl ether substituted PC in a biological sample at a first point in time and in at least one further biological sample at a later point in time and comparing said levels determined at the different time points.
10. The method of item 9, wherein the level which
   (i) decreases over time indicates that PPMS worsens in the patient,
   (ii) does not change over time indicates that PPMS does not worsen/is stable in the patient, or
   (iii) increases over time indicates that PPMS improves in the patient.
11. The method of item 10, wherein the patient receives or has received a treatment of PPMS.
12. The method of item 11, wherein the treatment of PPMS is selected from the group consisting of the administration of a drug, exercise training, mental training, and physical rehabilitation.
13. The method of any one of items 6 to 12, wherein the alkyl ether substituted PC is PC(O-20:0), preferably PC(O-16:0/4:0).
14. A method of determining the severity of primary progressive multiple sclerosis (PPMS) in a patient suspected of having PPMS comprising the step of:
   determining the level of an alkyl ether substituted PC in a biological sample from a patient suspected of having PPMS.
15. The method of item 14, wherein the level of the alkyl ether substituted PC is compared to a reference level of said alkyl ether substituted PC.
16. The method of item 15, wherein the reference level is the level determined by measuring
   at least one reference biological sample from
   at least one subject having PPMS.
17. The method of item 16, wherein
   the level of the alkyl ether substituted PC below the reference level indicates that the patient has a severe form of PPMS with a poor prognosis, or
   the level of the alkyl ether substituted PC above the reference level indicates that the patient has a mild form of PPMS with a good prognosis.
18. The method of any one of items 14 to 17, wherein the alkyl ether substituted PC is PC(O-20:0), preferably PC(O-16:0/4:0).
19. The method of any one of items 1 to 18, wherein the patient is a mammal, preferably a human.
20. The method of any one of items 1 to 19, wherein the biological sample is a body fluid sample.
21. The method of item 20, wherein the body fluid sample is selected from the group consisting of a blood sample and a urine sample.
22. The method of item 21, wherein the blood sample is a whole blood sample or a blood fraction sample.
23. The method of item 22, wherein the blood fraction sample is a blood cell fraction sample, a blood serum sample, or a blood plasma sample.
24. The method of any one of items 1 to 23, wherein the level of the at least one metabolite is determined by spectrometry and/or chromatography.
25. The method of item 24, wherein
   (i) the spectrometry is mass spectrometry (MS), preferably tandem mass spectrometry (MS/MS),
   (ii) the chromatography is liquid chromatography (LC) or affinity chromatography, or
   (iii) the chromatography is combined with spectrometry, preferably mass spectrometry (MS), and is more preferably liquid chromatography-mass spectrometry (LC-MS) and most preferably liquid chromatography-tandem mass spectrometry (LC-MS/MS).
26. Use of at least one metabolite for diagnosing primary progressive multiple sclerosis (PPMS) in a patient suspected of having PPMS,
   wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.
27. The use of item 26, wherein
   (i) LysoPE comprises an acyl group or an alkyl group or an 1Z-alkenyl group with 18 to 22 carbon atoms and with 0 to 4 double bonds,
      preferably, LysoPE is selected from the group consisting of LysoPE(18:1), LysoPE(18:2), and LysoPE(22:4),
   (ii) LysoPC comprises an acyl group or an alkyl group or an 1Z-alkenyl group with 16 to 20 carbon atoms and with 0 to 1 double bonds,
      preferably, LysoPC is selected from the group consisting of LysoPC(P-16:0), LysoPC(P-18:0), LysoPC(P-18:1), and LysoPC(20:1),
   (iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
      preferably, PE is PE(36:5),
      more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
   (iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
      preferably, PC is selected from the group consisting of PC(14:0), PC(O-20:0), PC(33:5), PC(35:5), PC(36:2), PC(36:3), and PC(44:12),
      more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6),
      even more preferably, PC is 1-(6-[3]-ladderane-hexanoyl)-2-(8-[3]-ladderane-octanyl)-sn-glycerophosphocholine,
   (v) hydroxy-octadecatrienoic acid is 2(R)-HOT, and/or
   (vi) octadecatrienoic acid is (6Z,9Z,12Z)-Octadecatrienoic acid.
28. Use of an alkyl ether substituted PC for determining the course of primary progressive multiple sclerosis (PPMS) in a patient having PPMS or for determining the severity of PPMS in a patient suspected of having PPMS.
29. The use of item 28, wherein the alkyl ether substituted PC is PC(O-20:0), preferably PC(O-16:0/4:0).
30. A kit comprising means for determining the level of at least one metabolite in a biological sample from a patient,
   wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.
31. The kit of item 30, wherein
   (i) LysoPE comprises an acyl group or an alkyl group or an 1Z-alkenyl group with 18 to 22 carbon atoms and with 0 to 4 double bonds,
      preferably, LysoPE is selected from the group consisting of LysoPE(18:1), LysoPE(18:2), and LysoPE(22:4),
   (ii) LysoPC comprises an acyl group or an alkyl group or an 1Z-alkenyl group with 16 to 20 carbon atoms and with 0 to 1 double bonds,
      preferably, LysoPC is selected from the group consisting of LysoPC(P-16:0), LysoPC(P-18:0), LysoPC(P-18:1), and LysoPC(20:1),
   (iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
      preferably, PE is PE(36:5),
      more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
   (iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
      preferably, PC is selected from the group consisting of PC(14:0), PC(O-20:0), PC(33:5), PC(35:5), PC(36:2), PC(36:3), and PC(44:12),
      more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6),
      even more preferably, PC is 1-(6-[3]-ladderane-hexanoyl)-2-(8-[3]-ladderane-octanyl)-sn-glycerophosphocholine,
   (v) hydroxy-octadecatrienoic acid is 2(R)-HOT, and/or
   (vi) octadecatrienoic acid is (6Z,9Z,12Z)-Octadecatrienoic acid.
32. The kit of items 30 or 31, wherein the kit is useful for conducting the methods according to any one of items 1 to 25.
33. The kit of any one of items 30 to 32, wherein the kit further comprises
   (i) a container, and/or
   (ii) a data carrier.
34. The kit of item 33, wherein the data carrier comprises instructions on how to carry out the methods according to any one of items 1 to 25.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following figures and examples are merely illustrative of the present invention and should not be construed to limit the scope of the invention as indicated by the appended claims in any way.

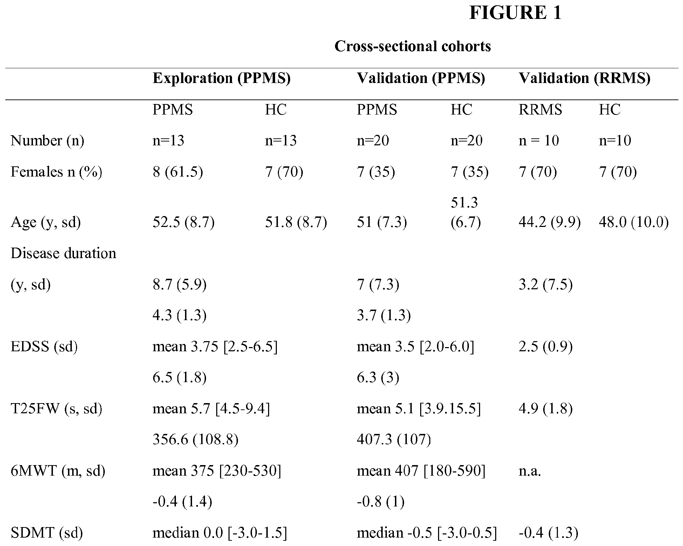

**Figure 1****: Demographic and clinical features of patients with multiple sclerosis and healthy controls in cross sectional PPMS and RRMS cohorts.** Abbreviations: PPMS = Primary Progressive Multiple Sclerosis, RRMS = Relapsing-Remitting Multiple Sclerosis, HC = healthy controls, EDSS = Expanded Disability Status Scale, T25FW = Timed-25-Foot Walk Test, 6MWT = Six Minute Walk Test, SDMT = Symbol Digit Modalities Test.

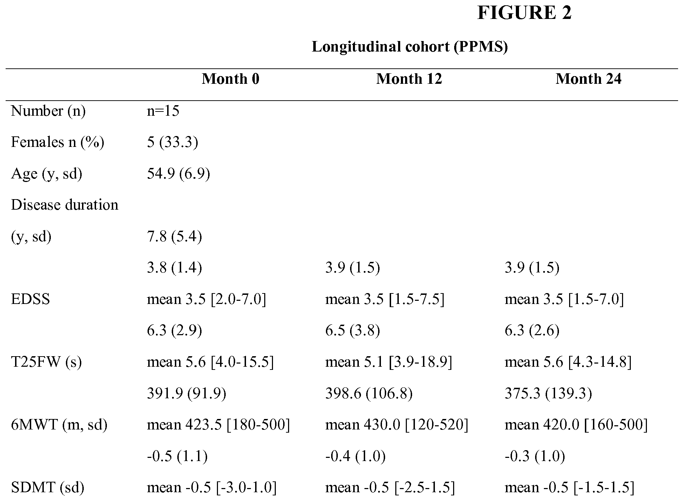

**Figure 2****: Demographic and clinical features of patients with multiple sclerosis and healthy controls in the longitudinal PPMS cohort.** Abbreviations: PPMS = Primary Progressive Multiple Sclerosis, EDSS = Expanded Disability Status Scale, T25FW = Timed-25-Foot Walk Test, 6MWT = Six Minute Walk Test, SDMT = Symbol Digit Modalities Test.

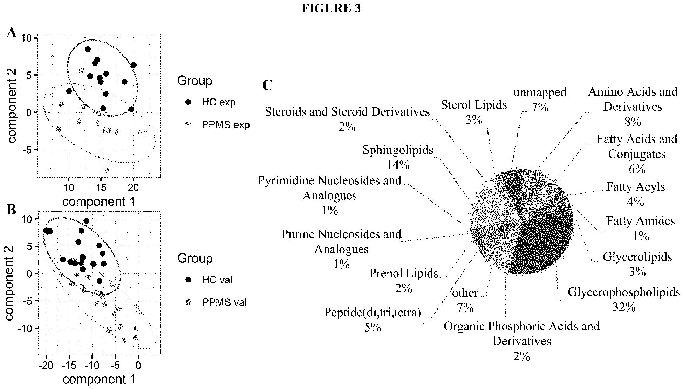

**Figure 3****: Separation of healthy controls and PPMS patients by partial least square discriminant analysis (PLS-DA) and distribution of total metabolites in human plasma samples.** (**A**) PLS-DA scores plot of all 534 metabolites which could be identified in HC exploration (exp) (black) and PPMS exp (grey) cohort. **(B)** PLS-DA scores plot of all 534 metabolite identified in the validation HC validation (black) and PPMS validation (grey) cohort. The ellipse assumes a multivariate distribution. **(C)** Pie chart illustrating the percentage of putatively identified metabolites from each metabolite class, classified according to KEGG, Lipidmaps and HMDB. In total 534 metabolites were analyzed.

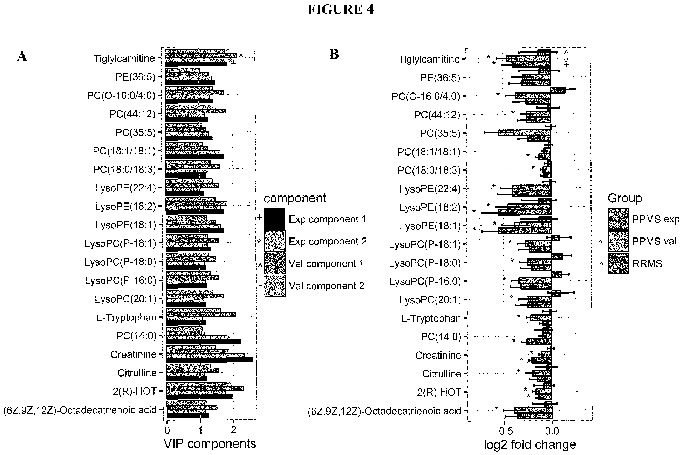

**Figure 4****: Extracted variable importance in the projection (VIP) component 1 plot for the exploration (exp) and validation (val) cohort and the corresponding log2 fold changes.** (**A**) Summary of 20 features (i.e. metabolites) in the dataset with VIP scores >1 for components 1 and 2. Component 1 in the val cohort (^), component 2 in the val cohort (-), component 1 in the exp cohort (+) and component 2 in the exp cohort (*). **(B)** log2 fold change values for metabolite set with calculated VIP values > 1 in both cohorts in comparison to healthy controls for PPMS patients. *significant change (Student's t-test α ≤ 0.05), log2 fold change > 0: up regulation, log2 fold change < 0: down regulation. (*): PPMS val, (+): PPMS exp, (^): RRMS.

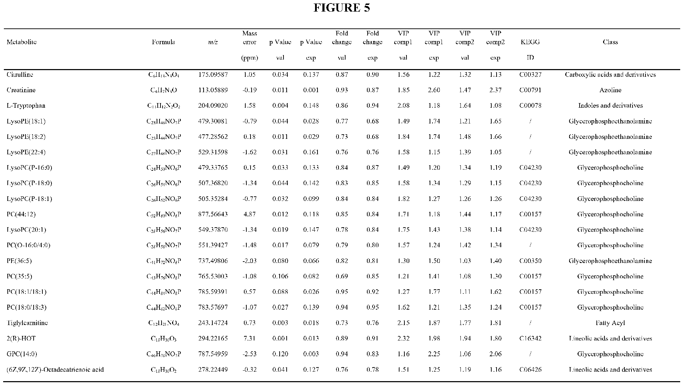

**Figure 5****: Metabolites with significant changes between HC and PPMS patients in the exp and val cohort.** Overlapping significant ions detected between HC and PPMS patients. Proposed metabolite: proposed metabolite for each ion. Proposed formula: Formulas obtained using m/z data with IDEOM. m/z: mass/charge ratio corrected for the mass of one proton. Mass error (ppm): [(m/z(observed)-m/z(theoretical)]*1E+6. Fold change: relative abundance of mean of corresponding ion in PPMS patients compared to the mean of HC. P value: value for unpaired Welch's t-test.

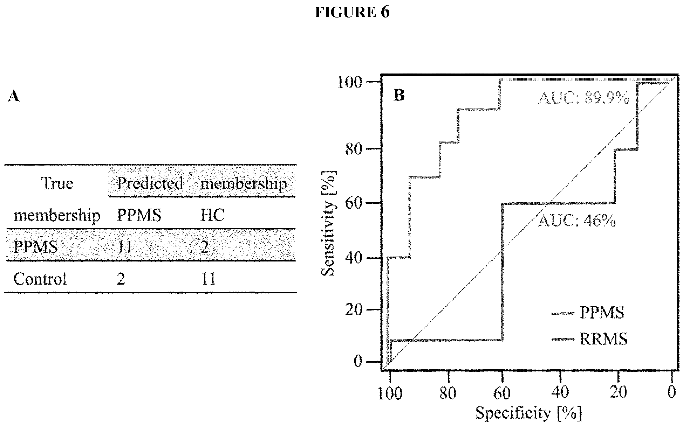

**Figure 6****: Representative partial least square (PLS) model for class membership determination and the corresponding receiver operating characteristics (ROC) curve. (A)** PLS confusion matrix for tested patients illustrating the true and false membership determined by the PLS model. **(B)** ROC curve of combined PLS model showing the true negative rate (specificity) vs. true positive rate (sensitivity): grey: representative PPMS PLS model, dark grey: representative PPMS PLS model applied against RRMS patients, grey line indicates 0 discrimination (AUC = 50%). AUC = area under the curve.

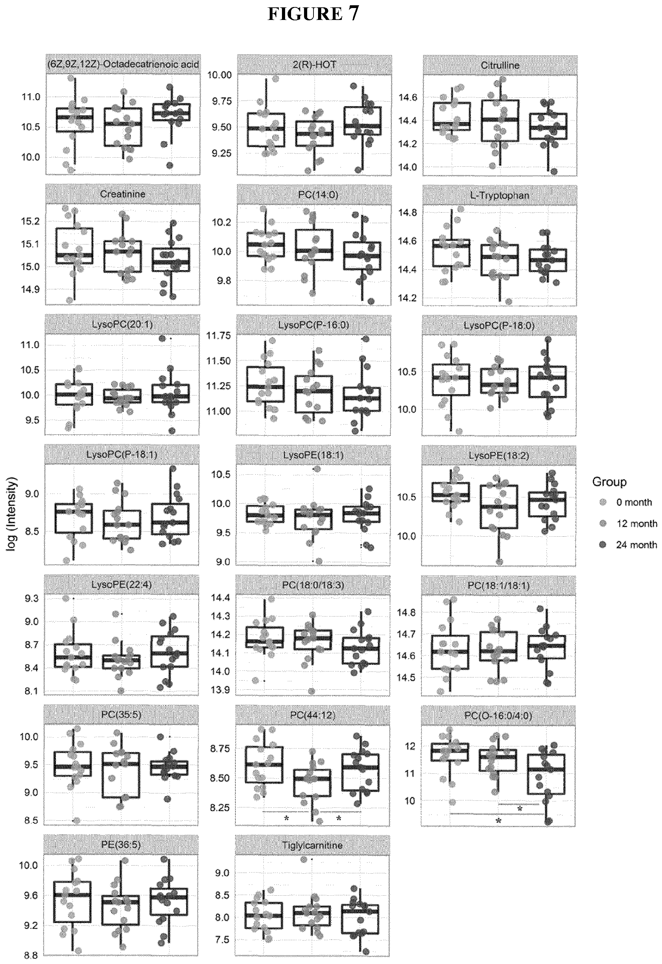

**Figure 7****: Box plot for potential PPMS marker during disease progression showing relative differences in abundance in each patient analyzed.** Light grey: 0 month, medium grey: 12 month, dark grey: 24 month. * Statistical significant change according to paired t-test statistics (α ≤ 0.01).

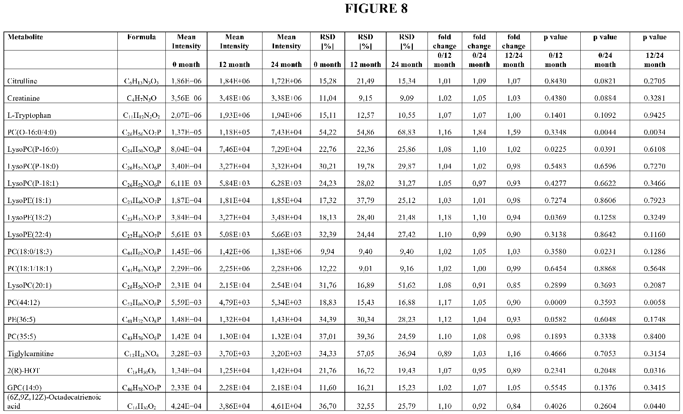

**Figure 8****: PPMS marker levels in the investigated progression samples** (0 month, 12 month and 24 month).

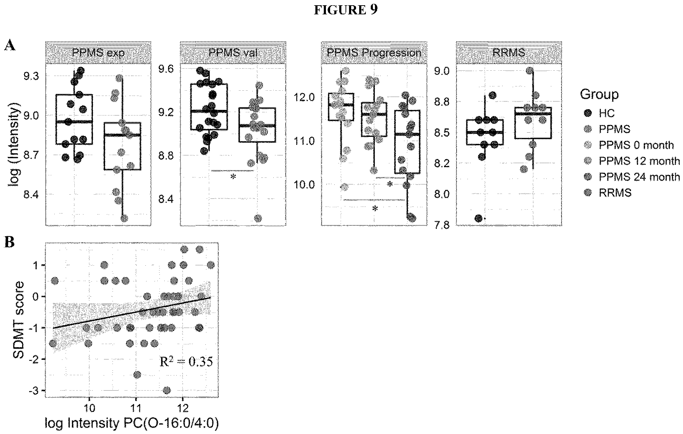

**Figure 9****: Box plot for glycerophospholipid PC(O-16:0/4:0) showing relative differences in abundance in each patient analyzed and correlation to SDMT scores.** (**A**) **PPMS exp:** decreased PC(O-16:0/4:0) levels in PPMS patients compared to healthy controls (HC), black: HC (n = 13), grey: PPMS patients (n = 13). **PPMS val:** PC(O-16:0/4:0) levels lower in PPMS patients compared to HC, dark grey: HC (n = 20), grey: PPMS patients (n = 20). **PPMS Progression:** Data from the progression cohort showing decreasing levels of PC(O-16:0/4:0) (0 month, 12 month and 24 month: n = 15), light grey: baseline (0 month), medium grey: PPMS 12 month, dark grey: PPMS 24 month, **RRMS:** black: HC (n=10), grey: RRMS patients (n=10), PC(O-16:0/4:0) appears to be elevated in RRMS patients compared to HC. **(B)** Spearman correlation of PC(O-16:0/4:0) values against SDMT scores during disease progression, grey area around the linear fit represents the 95% confidence interval.
**Figure 10****: Preferred metabolite combinations with their AUC values.**

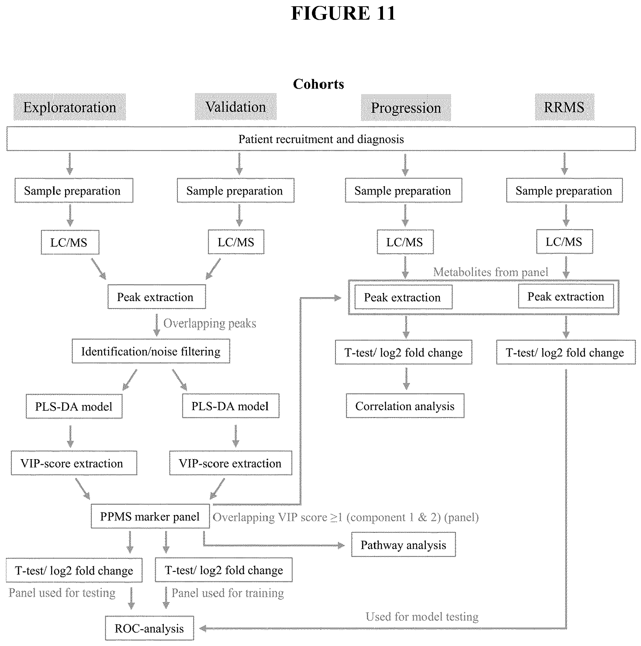

**Figure 11****: General workflow applied for the investigation of metabolic profiles of PPMS patients and their progression and differentiation to RRMS.**

### EXAMPLES

The examples given below are for illustrative purposes only and do not limit the invention described above in any way.

### Material and methods

### 1.1 Patient recruitment and diagnosis

PPMS and RRMS patients and HC were recruited at the University Medical Center Hamburg-Eppendorf. The participants underwent a clinical assessment and provided plasma samples. All MS patients fulfilled the revised 2010 McDonald criteria (Polman et al., 2011) and had an EDSS (Kurtzke, 1983) below 7.0. RRMS patients, PPMS patients and HC were recruited as a cross-sectional cohort, while additional PPMS patients were participating in a prospective observational longitudinal cohort study with annual visits and sampling of biomaterial. All participants provided written informed consent and the study was approved by the local investigational review board (Board of Physicians, Hamburg, No. PV4405 and PV3961). Several patients from the longitudinal cohort were also included in our cross sectional exploration PPMS cohort. The demographic and clinical features of patients with PPMS, RRMS and healthy controls are summarized in **Figure 1** **and** **Figure 2****.**

### 1.2 Human sample preparation

Peripheral blood was collected in S-Monovette® 9 ml, K3 EDTA, 92 x 16 mm test tubes (Sarstedt, 02.1066.001) centrifuged for 8 min at 1200 x g at 4 °C and centrifuged the supernatant at 4 °C for additional 10 min at 4300 x g. The supernatant was stored in liquid nitrogen until further analysis. Plasma metabolites were extracted using 90% MeOH and 10% H₂O spiked with internal standards with constant shaking for 15 min at 37 °C (1000 rpm) followed by centrifugation to remove the precipitate and the supernatant transferred into LC/MS vials.

### 1.3 LC/MS and MS/MS analysis

Modified hydrophilic interaction chromatography (HILIC) was employed in combination with high-resolution mass spectrometry (HRMS). Randomized samples were analyzed on an Agilent 1290 UHPLC system (Agilent, Santa Clara, USA) with a ZIC-HILIC column (10 cm x 2.1 mm, 3 µm, Sequant, Merck) coupled to a high-resolution 6540 QTOF/MS detector (Agilent, Santa Clara, USA) operated in both positive and negative ESI mode in a detection range of 50 to 1700 *m*/*z* at 2 GHz in extended dynamic range. The LC solvent consisted of (A) 95% 10 mM ammonium acetate with 5% acentonitrile pH 6 and (B) 95% acetonitrile with 5% 10 mM ammonium acetate with a multi-step gradient with 5% B from 0-1 min, then to 35% B at 8.5 min, to 95% B at 9.5 min kept constant until 12 min, to 5% B at 12.01 min and washing until 15 min with 5% B. The flow rate was kept constant at 300 µl/min. The total run time was 15 min, 1 µl of sample was injected and heated the column to 30 °C. The DualAJS ESI source was set to the following parameters: gas temperature 200 °C, drying gas 8 l/min, nebulizer 35 psi, sheath gas temp: 350°C, sheath gas flow 11 l/min, VCAp 3.5 kV and nozzle voltage of 0 V. Online calibration of the instrument was performed throughout the data acquisition using the Agilent ESI-TOF Reference Mass Solution Kit. MS/MS spectra were acquired in positive and negative ionization modes in a data dependent and targeted manner with fragmentation energies of 0 V, 10 V, 20 V and 40 V respectively. Precursor isolation windows varied between narrow (1.3 m/z), medium (4 m/z) and wide (9 m/z).

### 1.4 Metabolomics data analysis

Raw data was converted to mzXML and chromatogram peaks extracted with XCMS (Smith *et al.,* 2006), which were optimized by using the IPO R-package (Libiseller *et al.,* 2015). Mzmatch.R was used for peak filtering based on minimum detectable intensity (2000), peak shape filtering (codadw > 0.9) and for the annotation of related peaks (Scheltema *et al.,* 2011). Additional filtering was performed by excluding peaks with lower median peak intensities per group in biological samples compared to blanks (extraction solvent only). The remaining data was normalized based on multiple internal standards applying NOMIS (Sysi-Aho *et al.,* 2007) and CCMN (Redestig *et al.,* 2009) normalization. IDEOM software was used (http:// mzmatch.sourceforge.net/ideom.php) (Creek *et al.,* 2012) to eliminate noise and artefacts and for putative peak annotation by exact mass within ± 10 ppm against the Metabolomic Discoveries in-house metabolite library in negative and positive ESI mode, respectively. Retention time prediction was applied (Creek *et al.,* 2011) to aid metabolite annotation and identities confirmed by available authentic standards (validation level 1). MS/MS spectra were matched against online databases such as Metlin and MassBank (validation level 2) or against *in silico* fragmentation spectra (validation level 3) retrieved from Metfrag (Ruttkies *et al.,* 2016), CFM-ID (Allen *et al.,* 2014) and/or CSI:FingerID (Dührkop *et al.,* 2015) with precursor mass accuracy of 20 ppm and fragment accuracy of 0.01 Da. Quantification of each metabolite was calculated using the raw peak height.

### 1.5 Statistical analysis

Univariate statistical analyses utilized a Student's t-test (α = 0.05) and multivariate analyses utilized the metabolomics R package. Furthermore, "variable importance in the projection" (VIP) scores were computed and Student's t-test was applied to determine discriminatory variables in the dataset. The VIP is an estimation of the importance of each variable in the projection used in the partial least square discriminant analysis (PLS-DA) model (Seijo et al., 2013) as a quantitative estimation of the discriminatory power of each individual feature (Cho et al., 2008). Variables with a VIP score ≥1 were considered important in the PLS-DA model. PLS-DA computations were performed with the mixOmics R-package (Le Cao et al., 2009). Additional class discrimination and class membership prediction was performed for the subjects from different subgroups by training a PLS model with individuals from the reproduction cohort including 10-fold cross validation measures. The trained model was validated in the exploratory cohort and performance gauged by the measures AUC, sensitivity, specificity, 95% confidence intervals, accuracy (Acc), no information rate (NIR) and P value [Acc > NIR] of the calculated model. In addition, the model was used to determine specificity of the PPMS metabolic markers against marker levels in RRMS patients. The datasets was scaled prior to model building. For model prediction and AUC analysis, R-packages ROCR (Sing et al., 2005) and caret were used. Suitable markers were investigated, which enabled discrimination between HC and PPMS patients in the longitudinal cohort and time points 0 month (baseline), 12 months and 24 months utilizing paired t-test statistics (α = 0.01). Significant levels in MetaboAnalyst pathway analysis were based on hypergeometric test and the pathway impact values determined by relative-betweeness centrality (Xia et al., 2015).

### 1.6 Experimental design

For each sample a pseudonym was generated. Third party concealment of the origin of respective specimens (HC or MS) was achieved by using uniquely coded vials.

### Results

In order to identify metabolites that differentiate PPMS from healthy controls (HC), a comparative untargeted metabolomics approach in plasma of HC (n = 33) and PPMS patients (n = 33) was first employed, separated in an exploration cohort (n = 13 per group) and a validation cohort (n = 20 per group). Samples for each cohort were processed, extracted and measured independently (**Figure 11**).

After individual measurements peak extraction procedures on the raw data for the exploration (exp) and validation (val) cohort was combined to maximize the amount of overlapping mass peaks. Overall, 19,233 peaks in positive and 10,805 peaks in negative ionization mode, which were present in both the PPMS/HC exploration cohort and validation cohort, respectively. Successive noise filtering, putative peak annotation and combination of both ionization modes resulted in the nomination of 534 putative metabolites (Supplementary file "PPMS_metabolome.xlsx"). Identified metabolite classes included glycerophospholipids (32%), sphingolipids (14%), amino acids and derivatives (8%), unmapped (7%), fatty acids and conjugates (6%), peptides (di-,tri-,tetra-) (5%), fatty acyls (4%), sterol lipids (3%), glycerolipids (3%), steroids and steroid derivatives (2%), prenol lipids (2%), organic phosphoric acids and derivatives (2%), purine nucleosides and analogues (1%), pyrimidine nucleosides and analogues (1%) and fatty amides (1%). Additional metabolites in the group "other" included alkylamines, carbohydrates and carbohydrate conjugates, carboxylic acids and derivatives, cyclic alcohols and derivatives, hydroxy acids and derivatives, imidazopyrimidines, trisaccharides and xenobiotics (**Figure 3** **C**).

Next, the data were analyzed by a supervised, multivariate classification technique (partial least square discriminant analysis, PLS-DA) to assess the overall segregation of the samples for the exploration and validation cohort separately. PLS-DA was combined with univariate Student's t-test statistics to compare differences in the mean intensities per metabolite between HC and PPMS patients. This analysis showed that data from HC and PPMS samples could be separated into PPMS and HC reproducibly for the exploration and validation cohort (**Figure 3** **A and B**).

Based on the PLS-DA models, metabolites which contributed significantly to the differentiation between PPMS and HC in the exploration cohort and validation cohort were extracted by using VIP scores as a quantitative estimation of the discriminatory power of each individual metabolite. VIP values > 1 were considered to be significant. VIP scores were extracted for component 1 and 2 since these separated HC and PPMS almost to the same extend (**Figure 4** **A and B**). Overall 20 metabolites with a VIP score greater than 1 were reproducibly determined in both the PPMS exploration and validation cohorts (**Figure 4** **A,** Supplementary file "PPMS_Metabolome"). Of note, all of these metabolites were diminished in PPMS patients compared to HC and were also significantly altered in either one or both PPMS cohorts (Student's t-test α ≤ 0.05) (**Figure 4** **B**). The metabolites annotated as tiglylcarnitine (C12H21NO4), lysoPE(18:1) (C23H46NO7P), lysoPE(18:2) (C23H44NO7P) and 2(R)-HOT (C18H30O3) were significantly lower in PPMS patients compared to healthy controls in both the exploration and validation cohort (**Figure 5**).

In order to determine specificity of these 20 metabolites towards PPMS, these 20 metabolites were measured for PPMS in an independent cohort of 20 RRMS patients and 20 HC. In contrast to the PPMS cohort, lysoPC(P-18:1) (C26H52NO6P), lysoPC(P-18:0) (C26H54NO6P), lysoPC(P-16:0) (C24H50NO6P), PC(O-16:0/4:0) (C28H58NO7P) and lysoPC(P-20:1) (C28H56NO7P) were found to be elevated in RRMS patients compared to HC (**Figure 4** **B**). However, none of the 20 metabolites for PPMS were significantly changed in the RRMS cohort (Student's t-test α ≤ 0.05) which indicates specificity of our identified markers towards a PPMS plasma signature.

Subsequently, the 20 potential metabolic markers for PPMS were used to build a representative PLS classification model of two components to determine class membership of the measured samples. In order to ensure sufficient model training, the model with the larger validation cohort (Model parameter: accuracy (Acc): 85%, 95% confidence interval: (65%, 96%), p value: 2.7 x 10-4, sensitivity: 84.6%, specificity: 84.6%) was trained and tested against the blinded exploration cohort leading to 11/13 correctly identified PPMS patients and 11/13 correctly identified HC (**Figure 6** **A**)**.** Receiver operating characteristics (ROC) analysis of the training and testing model were used to build the corresponding ROC curve and could determine an AUC value of 89.9% (**Figure 6** **B**). The model was also compared against data obtained from RRMS patients and HC resulting in an AUC of 46% (**Figure 6** **B**). Thus, the resulting metabolite-based biomarker signature proved to be specific for PPMS.

To investigate the ability of the determined metabolite marker panel to monitor disease progression, a longitudinal PPMS cohort of 15 PPMS patients which have been monitored over a consecutive time period of 24 month was analyzed. At month 0 (baseline) patients were clinically monitored and plasma was sampled. The procedure was repeated after 12 months and 24 month.

Surprisingly, 18 of 20 determined metabolites showed no significant change between baseline (0 month), 12 month and 24 month progression (paired t-test α ≤ 0.01) (**Figure 7** **and** **Figure 8**). However, the glycerophospholipid annotated as PC(O-16:0/4:0) showed a significant reduction when comparing 12 month and 24 month values (p-value = 0.0034 (paired t-test), fold change (12 month/24 month) = 1.59) or baseline (0 month) and 24 month values (p-value = 0.0044 (paired t-test), fold change (0 month/24 month) = 1.84). This glycerophospholipid also showed reduced levels in PPMS patients compared to healthy controls in the validation and exploration cohort and was found to be elevated in RRMS compared to HC (**Figure 9 A)****.** In addition PC(44:12) levels were significantly lowered in 12 months compared to baseline (paired t-test p-value = 0.0009; fold change 0 month/12 month = 1.17) and also lowered in 12 month patients compared to the 24 month time point (paired t-test p-value = 0.0058; fold change 12 month/24 month = 0.90) while levels at 24 months were similar to baseline. In conclusion, the data show no clear trend for the determined PPMS metabolite-based biomarker signature in disease progression with exception of PC(O-16:0/4:0). In addition, spearman correlation measures were used to correlate PC(O-16:0/4:0) values to the corresponding EDSS scores (disability), SDMT (cognition) and walking test performances (**Figure 2**). The best correlation could be achieved using SDMT (cognition) scores with a correlation coefficient of 0.35 (EDSS: 0.13, 6MWT: -0.3, T25FW: 0.18) (**Figure 9** **B**).

### Discussion

Primary progressive multiple sclerosis is a highly variable neurodegenerative disease, and compared to other MS subtypes, it is associated with a poor prognosis and continuous accumulation of neurological symptoms and disability (Antel *et al.,* 2012). Currently, no reliable molecular biomarker-based diagnosis is available. Hence, novel and easier diagnostic tools for PPMS are needed. The untargeted metabolomics approach presented here lead to the identification of 534 blood plasma metabolites in both HC and PPMS patients. A PLS-DA model based on their quantification showed a clear separation between the investigated PPMS patients and healthy controls, which was attributed to a total of 20 metabolites. These findings were validated in two independent cohorts and showed a predictive power of 89.9% AUC. The results support the hypothesis that metabolomic patterns can be used to discriminate HC from PPMS patients as 11 of 13 PPMS patients were correctly identified by this model. The established model also shows a high specificity for PPMS compared to RRMS patients as the calculated AUC was only 46% with no significant results in RRMS. A medication bias in the study presented was reduced by comparing patients with relatively heterogeneous treatments regimes in our clinical PPMS and RRMS cohorts. Based on the medication information it was not possible to build a PLS model which indicates that medication was no confounding factor in the determined marker profile. Moreover, sex bias in MS (Voskuhl and Gold, 2012) was reduced by comparing equal sex distributions in the different categories (HC, PPMS, RRMS and longitudinal cohort) (**Figure 1** **and** **2**). Furthermore, an age-related bias in the analysis was tested (age difference not significant according to type I ANOVA α = 0.05) and the comparison was performed on age-matched cohorts.

In addition, almost all determined metabolite markers that discriminate between HC and PPMS patients showed no significant change over the investigated time course (24 month). However, the lipid annotated as PC(O-16:0/4:0) is a notable exception and is significantly reduced in disease progression between the baseline (0 month) and 24 month and between 24 month and 12 month. These values could also be positively correlated to Symbol Digit Modalities Test measures which is indicating disease impairment over PPMS progression. Elevated levels (not significant) of PC(O-16:0/4:0) in RRMS patients compared to healthy controls could indicate a different trend of this marker in RRMS, which strengthens its specificity for PPMS and PPMS progression. In addition, significantly lowered levels of PC(44:12) were detected between 0 month and 12 month and between 12 month and 24 month. However this lipid does not show a clear trend since levels in the 24 month patients were detected on baseline level.

In conclusion, metabolic profiling of plasma was found to be a suitable technology for noninvasive diagnosis of PPMS patients. The results of this study disclose biomarkers for PPMS diagnosis and progression with promising insights in altered glycerophospholipid metabolism of PPMS patients with a high specificity compared to RRMS. **Figure 10** shows, for example, specific biomarker combinations for diagnosing PPMS having a high AUC value.

### REFERENCES

Allen F, Pon A, Wilson M, Greiner R, Wishart D. CFM-ID: a web server for annotation, spectrum prediction and metabolite identification from tandem mass spectra. Nucleic Acids Res. 2014; 42: W94-9.
Antel J, Antel S, Caramanos Z, Arnold DL, Kuhlmann T. Primary progressive multiple sclerosis: part of the MS disease spectrum or separate disease entity? Acta Neuropathol 2012; 123
Bhargava P, Calabresi PA. Metabolomics in multiple sclerosis. Mult. Scler. 2016; 22: 451-460. Cocco E, Murgia F, Lorefice L, Barberini L, Poddighe S, Frau J, et al. (1)H-NMR analysis provides a metabolomic profile of patients with multiple sclerosis. Neurol. Neuroimmunol. neuroinflammation 2016; 3: e185.
Compston A, Coles A. Multiple sclerosis. Lancet (London, England) 2008; 372: 1502-1517.
Contrepois K, Jiang L, Snyder M. Optimized Analytical Procedures for the Untargeted Metabolomic Profiling of Human Urine and Plasma by Combining Hydrophilic Interaction (HILIC) and Reverse-Phase Liquid Chromatography (RPLC)-Mass Spectrometry. Mol. Cell. Proteomics 2015; 14: 1684-1695.
Correale J, Gaitán MI, Ysrraelit MC, Fiol MP. Progressive multiple sclerosis: from pathogenic mechanisms to treatment. Brain 2016.
Creek DJ, Jankevics A, Breitling R, Watson DG, Barrett MP, Burgess KE V. Toward Global Metabolomics Analysis with Hydrophilic Interaction Liquid Chromatography-Mass Spectrometry: Improved Metabolite Identification by Retention Time Prediction. Anal. Chem. 2011; 83: 8703-8710.
Creek DJ, Jankevics A, Burgess KE V, Breitling R, Barrett MP. IDEOM: an Excel interface for analysis of LC-MS-based metabolomics data. Bioinformatics 2012; 28: 1048-1049.
Dührkop K, Shen H, Meusel M, Rousu J, Böcker S. Searching molecular structure databases with tandem mass spectra using CSI:FingerID. Proc. Natl. Acad. Sci. 2015; 112 : 12580-12585.
Fitzner D, Simons M. Chronic Progressive Multiple Sclerosis - Pathogenesis of Neurodegeneration and Therapeutic Strategies. Curr. Neuropharmacol. 2010; 8: 305-315. Floegel A, Drogan D, Wang-Sattler R, Prehn C, Illig T, Adamski J, et al. Reliability of serum metabolite concentrations over a 4-month period using a targeted metabolomic approach. PLoS One 2011; 6: e21103.
Fox RJ, Thompson A, Baker D, Baneke P, Brown D, Browne P, et al. Setting a research agenda for progressive multiple sclerosis: The International Collaborative on Progressive MS. Mult. Scler. 2012; 18: 1534-1540.
Friese MA, Schattling B, Fugger L. Mechanisms of neurodegeneration and axonal dysfunction in multiple sclerosis. Nat. Rev. Neurol. 2014; 10: 225-238.
Hassan-Smith G, Wallace GR, Douglas MR, Sinclair AJ. The role of metabolomics in neurological disease. J. Neuroimmunol. 2016; 248: 48-52.
Koch M, Kingwell E, Rieckmann P, Tremlett H. The natural history of primary progressive multiple sclerosis. Neurology 2009; 73: 1996-2002.
Libiseller G, Dvorzak M, Kleb U, Gander E, Eisenberg T, Madeo F, et al. IPO: a tool for automated optimization of XCMS parameters. BMC Bioinformatics 2015; 16: 1-10.
Poisson LM, Suhail H, Singh J, Datta I, Denic A, Labuzek K, et al. Untargeted Plasma Metabolomics Identifies Endogenous Metabolite with Drug-like Properties in Chronic Animal Model of Multiple Sclerosis. J. Biol. Chem. 2015; 290: 30697-30712.
Polman CH, Reingold SC, Banwell B, Clanet M, Cohen JA, Filippi M, et al. Diagnostic criteria for multiple sclerosis: 2010 Revisions to the McDonald criteria. Ann. Neurol. 2011; 69: 292-302.
Polman CH, Reingold SC, Edan G, Filippi M, Hartung HP, Kappos L. Diagnostic criteria for multiple sclerosis: 2005 revisions to the "McDonald Criteria". Ann Neurol 2005; 58.
Redestig H, Fukushima A, Stenlund H, Moritz T, Arita M, Saito K, et al. Compensation for Systematic Cross-Contribution Improves Normalization of Mass Spectrometry Based Metabolomics Data. Anal. Chem. 2009; 81: 7974-7980.
Reinke SN, Broadhurst DL, Sykes BD, Baker GB, Catz I, Warren KG, et al. Metabolomic profiling in multiple sclerosis: insights into biomarkers and pathogenesis. Mult. Scler. 2014; 20: 1396-1400.
Ruttkies C, Schymanski EL, Wolf S, Hollender J, Neumann S. MetFrag relaunched: incorporating strategies beyond in silico fragmentation. J. Cheminform. 2016; 8: 3. Scheltema RA, Jankevics A, Jansen RC, Swertz MA, Breitling R. PeakML/mzMatch: a file format, Java library, R library, and tool-chain for mass spectrometry data analysis. Anal. Chem. 2011; 83: 2786-2793.
Smith CA, Want EJ, O'Maille G, Abagyan R, Siuzdak G. XCMS: Processing Mass Spectrometry Data for Metabolite Profiling Using Nonlinear Peak Alignment, Matching, and Identification. Anal. Chem. 2006; 78: 779-787.
Smolinska A, Blanchet L, Buydens LMC, Wijmenga SS. NMR and pattern recognition methods in metabolomics: from data acquisition to biomarker discovery: a review. Anal. Chim. Acta 2012; 750: 82-97.
Stellmann J-P, Neuhaus A, Lederer C, Daumer M, Heesen C. Validating Predictors of Disease Progression in a Large Cohort of Primary-Progressive Multiple Sclerosis Based on a Systematic Literature Review. PLoS One 2014; 9: e92761.
Sysi-Aho M, Katajamaa M, Yetukuri L, Oresic M. Normalization method for metabolomics data using optimal selection of multiple internal standards. BMC Bioinformatics 2007; 8: 93.
Tremlett H, Paty D, Devonshire V. The natural history of primary progressive MS in British Columbia, Canada. Neurology 2005; 65: 1919-1923.
Voskuhl RR, Gold SM. Sex-related factors in multiple sclerosis susceptibility and progression. Nat Rev Neurol 2012; 8: 255-263.
Want EJ, Masson P, Michopoulos F, Wilson ID, Theodoridis G, Plumb RS, et al. Global metabolic profiling of animal and human tissues via UPLC-MS. Nat. Protoc. 2013; 8: 17-32.
Zhou B, Xiao JF, Tuli L, Ressom HW. LC-MS-based metabolomics. Mol. Biosyst. 2012; 8: 470-481.

## Claims

1. A method of diagnosing primary progressive multiple sclerosis (PPMS) in a patient suspected of having PPMS comprising the step of:
determining the level of at least one metabolite in a biological sample from a patient suspected of having PPMS,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

2. The method of claim 1, wherein the level of the at least one metabolite is compared to a reference level of said at least one metabolite,
wherein preferably the reference level is the level determined by measuring at least one reference biological sample from at least one healthy subject, and
wherein more preferably the level of the at least one metabolite below the reference level indicates that the patient has PPMS.

3. The method of claims 1 or 2, wherein
(i) LysoPE comprises an acyl group or an alkyl group or an 1Z-alkenyl group with 18 to 22 carbon atoms and with 0 to 4 double bonds,
preferably, LysoPE is selected from the group consisting of LysoPE(18:1), LysoPE(18:2), and LysoPE(22:4),
(ii) LysoPC comprises an acyl group or an alkyl group or an 1Z-alkenyl group with 16 to 20 carbon atoms and with 0 to 1 double bonds,
preferably, LysoPC is selected from the group consisting of LysoPC(P-16:0), LysoPC(P-18:0), LysoPC(P-18:1), and LysoPC(20:1),
(iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
preferably, PE is PE(36:5),
more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
(iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
preferably, PC is selected from the group consisting of PC(14:0), PC(O-20:0), PC(33:5), PC(35:5), PC(36:2), PC(36:3), and PC(44:12),
more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6),
even more preferably, PC is 1-(6-[3]-ladderane-hexanoyl)-2-(8-[3]-ladderane-octanyl)-sn-glycerophosphocholine,
(v) hydroxy-octadecatrienoic acid is 2(R)-HOT, and/or
(vi) octadecatrienoic acid is (6Z,9Z,12Z)-Octadecatrienoic acid.

4. A method of determining the course of primary progressive multiple sclerosis (PPMS) in a patient having PPMS comprising the step of:
determining the level of an alkyl ether substituted PC in a biological sample from a patient having PPMS.

5. The method of claim 4, wherein the level of the alkyl ether substituted PC is compared to a reference level of said alkyl ether substituted PC,
wherein preferably the reference level is the level determined by measuring at least one reference biological sample from
at least one healthy subject, or
at least one subject having PPMS.

6. The method of claims 4 or 5, wherein said determining comprises determining the level of the alkyl ether substituted PC in a biological sample at a first point in time and in at least one further biological sample at a later point in time and comparing said levels determined at the different time points.

7. The method of claim 6, wherein the level which
(i) decreases over time indicates that PPMS worsens in the patient,
(ii) does not change over time indicates that PPMS does not worsen/is stable in the patient, or
(iii) increases over time indicates that PPMS improves in the patient.

8. A method of determining the severity of primary progressive multiple sclerosis (PPMS) in a patient suspected of having PPMS comprising the step of:
determining the level of an alkyl ether substituted PC in a biological sample from a patient suspected of having PPMS.

9. The method of claim 8, wherein the level of the alkyl ether substituted PC is compared to a reference level of said alkyl ether substituted PC,
wherein preferably the reference level is the level determined by measuring at least one reference biological sample from
at least one subject having PPMS, and
wherein more preferably the level of the alkyl ether substituted PC below the reference level indicates that the patient has a severe form of PPMS with a poor prognosis, or the level of the alkyl ether substituted PC above the reference level indicates that the patient has a mild form of PPMS with a good prognosis.

10. Use of at least one metabolite for diagnosing primary progressive multiple sclerosis (PPMS) in a patient suspected of having PPMS,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

11. The use of claim 10, wherein
(i) LysoPE comprises an acyl group or an alkyl group or an 1Z-alkenyl group with 18 to 22 carbon atoms and with 0 to 4 double bonds,
preferably, LysoPE is selected from the group consisting of LysoPE(18:1), LysoPE(18:2), and LysoPE(22:4),
(ii) LysoPC comprises an acyl group or an alkyl group or an 1Z-alkenyl group with 16 to 20 carbon atoms and with 0 to 1 double bonds,
preferably, LysoPC is selected from the group consisting of LysoPC(P-16:0), LysoPC(P-18:0), LysoPC(P-18:1), and LysoPC(20:1),
(iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
preferably, PE is PE(36:5),
more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
(iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
preferably, PC is selected from the group consisting of PC(14:0), PC(O-20:0), PC(33:5), PC(35:5), PC(36:2), PC(36:3), and PC(44:12),
more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6),
even more preferably, PC is 1-(6-[3]-ladderane-hexanoyl)-2-(8-[3]-ladderane-octanyl)-sn-glycerophosphocholine,
(v) hydroxy-octadecatrienoic acid is 2(R)-HOT, and/or
(vi) octadecatrienoic acid is (6Z,9Z,12Z)-Octadecatrienoic acid.

12. Use of an alkyl ether substituted PC for determining the course of primary progressive multiple sclerosis (PPMS) in a patient having PPMS or for determining the severity of PPMS in a patient suspected of having PPMS.

13. The use of claim 12, wherein the alkyl ether substituted PC is PC(O-20:0), preferably PC(O-16:0/4:0).

14. A kit comprising means for determining the level of at least one metabolite in a biological sample from a patient,
wherein the at least one metabolite is selected from the group consisting of citrulline, creatinine, L-tryptophan, LysoPE, LysoPC, PE, PC, tiglylcarnitine, hydroxy-octadecatrienoic acid, and octadecatrienoic acid.

15. The kit of claim 14, wherein
(i) LysoPE comprises an acyl group or an alkyl group or an 1Z-alkenyl group with 18 to 22 carbon atoms and with 0 to 4 double bonds,
preferably, LysoPE is selected from the group consisting of LysoPE(18:1), LysoPE(18:2), and LysoPE(22:4),
(ii) LysoPC comprises an acyl group or an alkyl group or an 1Z-alkenyl group with 16 to 20 carbon atoms and with 0 to 1 double bonds,
preferably, LysoPC is selected from the group consisting of LysoPC(P-16:0), LysoPC(P-18:0), LysoPC(P-18:1), and LysoPC(20:1),
(iii) PE comprises two groups independently from each other selected from the group consisting of an acyl group, an alkyl group and an 1Z-alkenyl group, wherein said two groups have a total number of between 14 and 44 carbon atoms and a total number of between 0 and 5 double bonds,
preferably, PE is PE(36:5),
more preferably, PE is selected from the group consisting of PE(14:1/22:4), PE(18:1/18:4), PE(18:2/18:3), PE(18:3/18:2), PE(18:4/18:1), PE(20:4/16:1), PE(20:5/16:0), PE(22:4/14:1), PE(16:1/20:4), and PE(16:0/20:5),
(iv) PC comprises two groups independently from each other selected from the group consisting of an acyl group, an alkyl group and an 1Z-alkenyl group, wherein said two groups have a total number of between 14 and 44 carbon atoms and a total number of between 0 and 12 double bonds,
preferably, PC is selected from the group consisting of PC(14:0), PC(O-20:0), PC(33:5), PC(35:5), PC(36:2), PC(36:3), and PC(44:12),
more preferably, PC is selected from the group consisting of PC(6:0/8:0), PC(O-16:0/4:0), PC(13:0/20:5), PC(15:1/18:4), PC(18:4/15:1), PC(20:5/13:0), PC(18:1/18:1), PC(18:0/18:3), PC(15:0/20:5), PC(17:1/18:4), PC(15:1/20:4), PC(17:2/18:3), PC(18:3/17:2), PC(18:4/17:1), PC(20:4/15:1), PC(20:5/15:0), and PC(22:6/22:6),
even more preferably, PC is 1-(6-[3]-ladderane-hexanoyl)-2-(8-[3]-ladderane-octanyl)-sn-glycerophosphocholine,
(v) hydroxy-octadecatrienoic acid is 2(R)-HOT, and/or
(vi) octadecatrienoic acid is (6Z,9Z,12Z)-Octadecatrienoic acid.
